# EUROPEAN PATENT SPECIFICATION

(11) **EP 2 663 580 B1**
(45) Date of publication and mention of the grant of the patent: **07.12.2016**
(21) Application number: 12701463.7
(22) Date of filing: 10.01.2012
(51) Int. Cl.: C07K 16/30, A61K 39/395, C07K 16/28, A61K 39/00

(54) **COMBINATION THERAPY INCLUDING TUMOR ASSOCIATED ANTIGEN BINDING ANTIBODIES**
KOMBINATIONSTHERAPIE MIT TUMORASSOZIIERTEN, ANTIGENBINDENDEN ANTIKÖRPERN
POLYTHÉRAPIE COMPRENANT DES ANTICORPS SE LIANT AUX ANTIGÈNES ASSOCIÉS AUX TUMEURS

(30) Priority: 10.01.2011 EP 11150527
(43) Date of publication of application: 20.11.2013
(73) Proprietor: CT Atlantic Ltd., 8952 Schlieren (CH); Universität Zürich, 8006 Zürich (CH)
(72) Inventor: ESSLINGER, Christoph, CH-8032 Zürich (CH); KNUTH, Alexander, CH-8002 Zürich (CH); TREDER, Martin, CH-8049 Zürich (CH); VAN DEN BROEK, Maries, CH-8032 Zürich (CH); JAEGER, Elke, 55246 Mainz-Kostheim (DE)
(74) Representative: Bühler, Dirk
(86) International application number: PCT/EP2012/050295
(87) International publication number: WO 2012/095412

(56) References cited:
- WO-A1-2008/110372
- WO-A2-2005/000870
- WO-A2-2005/032475
- WO-A2-2005/105139
- US-B1- 6 252 052
- LOHMANN CHRISTINA M ET AL: "Primary malignant melanoma of the oesophagus: a clinical and pathological study with emphasis on the immunophenotype of the tumours for melanocyte differentiation markers and cancer/testis antigens", MELANOMA RESEARCH, LIPPINCOTT WILLIAMS & WILKINS, US, vol. 13, no. 6, 1 December 2003 (2003-12-01), pages 595-601, XP008135853, ISSN: 0960-8931
- SCHULTZ-THATER E ET AL: "NY-ESO-1 tumour associated antigen is a cytoplasmic protein detectable by specific monoclonal antibodies in cell lines and clinical specimens.", BRITISH JOURNAL OF CANCER JUL 2000 LNKD- PUBMED:10901371, vol. 83, no. 2, July 2000 (2000-07), pages 204-208, XP002636527, ISSN: 0007-0920
- KAWABATA RYOHEI ET AL: "Antibody response against NY-ESO-1 in CHP-NY-ESO-1 vaccinated patients.", INTERNATIONAL JOURNAL OF CANCER. JOURNAL INTERNATIONAL DU CANCER 15 MAY 2007 LNKD- PUBMED:17278093, vol. 120, no. 10, 15 May 2007 (2007-05-15) , pages 2178-2184, XP002490651, ISSN: 0020-7136
- AOKI MASATOSHI ET AL: "Antibody responses against NY-ESO-1 and HER2 antigens in patients vaccinated with combinations of cholesteryl pullulan (CHP)-NY-ESO-1 and CHP-HER2 with OK-432.", VACCINE 16 NOV 2009 LNKD- PUBMED:19761832, vol. 27, no. 49, 16 November 2009 (2009-11-16), pages 6854-6861, XP002636529, ISSN: 1873-2518
- VALMORI DANILA ET AL: "Identification of B cell epitopes recognized by antibodies specific for the tumor antigen NY-ESO-1 in cancer patients with spontaneous immune responses.", CLINICAL IMMUNOLOGY (ORLANDO, FLA.) OCT 2005 LNKD- PUBMED:15994128, vol. 117, no. 1, October 2005 (2005-10), pages 24-30, XP002636530, ISSN: 1521-6616
- GNJATIC SACHA ET AL: "NY-ESO-1: REVIEW OF AN IMMUNOGENIC TUMOR ANTIGEN", ADVANCES IN CANCER RESEARCH, ACADEMIC PRESS, US, 1 January 2006 (2006-01-01), pages 1-30, XP008073732, ISSN: 0065-230X, DOI: DOI:10.1016/S0065-230X(06)95001-5

## Description

### FIELD OF THE INVENTION

The present invention relates to combinations of tumor associated antigen binding antibodies or binding fragments thereof and compounds capable of activating the immune system. The present invention further relates to the use of such combinations for treating diseases, in particular hyper-proliferative diseases and methods for treating diseases, in particular hyper-proliferative diseases with such combinations.

### BACKGROUND OF THE INVENTION

In cancer therapy, it is a general aim to treat the afflicted tissues as efficiently and selectively as possible. Therapeutic monoclonal antibodies have been conceived as a class of pharmaceutically active agents which should allow tumor selective treatment by targeting tumor selective antigens or epitopes.

However, in some cancers, for example those associated with human growth factor receptors such as HER-2 R or EGFR, epitopes targeted by therapeutic antibodies are also found on normal tissues explaining adverse side effects upon antibody administration or peripheral sink effects in the pharmacokinetic behavior of such antibodies.

The analogous situation holds true by applying systemically active immune-stimulatory drugs or antibodies applied to stimulate a natural immune response to fight cancer. Such immune-stimulants are for example activators of the innate immune system such as activators of TLR-7 or TLR-9 receptors.

Monoclonal antibodies have nevertheless enjoyed increasing acceptance as therapeutic tools for treating cancer over the past decades. The advent of chimeric antibodies and humanized antibodies significantly contributed to the success of monoclonal therapeutic antibodies as these second and third generation monoclonal antibodies showed improved side-effect profiles compared to the original mice-derived monoclonal antibodies in view of their reduced immunogenicity.

Despite the proven therapeutic efficacy of humanized antibodies, there is an interest in fully human antibodies. However, production thereof is still prone to technical difficulties. For example, generating fully human antibodies in mice in which the antibody encoding genomic regions have been replaced by the human counterpart remains burdensome. Alternative approaches such as phage display lack the natural variability and complexity of the human immune system.

There is thus continuing need for therapeutic monoclonal antibodies which allow for a (tumor) localized mode of action and which have an increased chance of meeting regulatory approval. Moreover, there is a wish for cancer therapies in general which allow for improved efficacy.

### OBJECTIVES AND SUMMARY OF THE INVENTION

It is an objective of the present invention to provide combinations of pharmaceutically active agents which can be used as therapeutic tool for treating human diseases including hyper-proliferative diseases such as cancer. In particular, it is an objective of the present invention to provide combinations of pharmaceutically active agents which can be used to selectively treat hyper-proliferative diseases by ensuring a localized immune reaction in the afflicted tissue.

It is a further objective of the present invention to provide antibodies which can be used as therapeutic tool for treating human diseases including hyper-proliferative diseases such as cancer. In particular, it is an objective of the present invention to provide human antibodies which can be used to selectively treat hyper-proliferative diseases by ensuring a localized immune reaction in the afflicted tissue.

Further, it is an objective of the present invention to provide methods of treating patients suffering e.g. from hyper-proliferative diseases such as cancer by making use of such combinations of pharmaceutically active agents and antibodies.

These and other objectives as they will become apparent from the ensuing description hereinafter are solved by the subject matter of the independent claims. Some of the preferred embodiments of the present invention form the subject matter of the dependent claims. Yet other embodiments of the present invention may be taken from the ensuing description.

In a first aspect the invention relates to a pharmaceutical composition comprising at least one tumor associated antigen (TAA) binding antibody or binding fragment thereof and at least one compound capable of activating the immune system.

As will become apparent from the ensuing description, such TAA binding antibodies or binding fragments thereof preferably bind to CT antigens with NY-ESO-1. Tumor-specific antibodies binding tumor-associated antigen NY-ESO-1 are desdribed in WO 2008/110372 A1. Such antibodies or binding fragments thereof may be monoclonal chimeric, humanized or human antibodies or binding fragments thereof. Patient-derived, human, monoclonal antibodies may be preferred.

Exemplary NY-ESO-1 binding antibodies or fragments thereof may comprise a variable heavy chain and/or a variable light chain of the exemplary antibodies 12D7, 12D7*, 31E4, 30D6, 15B12, 22A1, 1H12, 10E1 or 1D4 or a variable heavy chain and/or a variable light chain having at least 80% sequence identity with the variable heavy chain and/or variable light chain of the exemplary antibodies 12D7, 12D7*, 31E4, 30D6, 15B12, 22A1, 1H12, 10E1 or 1D4.

Other exemplary NY-ESO-1 binding antibodies or fragments thereof may comprise the complementary determining regions (CDRs) of the exemplary antibodies 12D7, 12D7*, 31E4, 30D6, 15B12, 22A1, 1H12, 10E1 or 1D4 within their variable heavy chain and/or variable light chain. Such antibodies may also comprise CDRs within their variable heavy chain and/or variable light chain having at least 80% sequence identity with the CDRs of the exemplary antibodies 12D7, 12D7*, 31E4, 30D6, 15B12, 22A1, 1H12, 10E1 or 1D4.

As will become apparent from the ensuing description, compounds capable of activating the immune response may preferably be selected from at least one natural stimulant or at least co-stimulant of the immune system, agonistic activator of natural stimulants or at least co-stimulants of the immune system or at least one antagonistic effector of natural inhibitors or at least co-inhibitors of the immune system as described hereinafter. Some preferred exemplary representatives are CD40L, anti-CD40 agonistic antibodies such as CP-870,893 and SGN-40 and anti-CTLA4 antagonistic antibodies such as Tremelimumab and Ipilimumab.

Exemplary embodiments thus relate to pharmaceutical compositions comprising (i) the afore-mentioned NY-ESO-1 binding antibodies or fragments thereof, and (ii) CD40L, or anti-CD40 agonistic antibodies such as CP-870,893 and SGN-40, or anti-CTLA4 antagonistic antibodies such as Tremelimumab and Ipilimumab.

An embodiment describes that the pharmaceutical composition may comprise (i) a TAA binding antibody or binding fragment as described above, (ii) at least one natural stimulant or at least co-stimulant of the immune system, or at least one agonistic activator of natural stimulants or at least co-stimulants of the immune system and (iii) at least one antagonistic effector of natural inhibitors or at least co-inhibitors of the immune system as described hereinafter. Some preferred exemplary embodiments relate to pharmaceutical compositions comprising (i) the afore-mentioned NY-ESO-1 binding antibodies or fragments thereof, (ii) CD40L or anti-CD40 agonistic antibodies such as CP-870,893 or SGN-40 and (iii) anti-CTLA4 antagonistic antibodies such as Tremelimumab and Ipilimumab.

In a second aspect of the invention, the aforementioned pharmaceutically active agents, i.e. the TAA binding antibodies or fragments thereof and the compounds capable of stimulating the immune system are not combined within a single pharmaceutical composition but actually are presented in form of a kit consisting of various pharmaceutical compositions wherein the active agents are split at least to some extent between the various pharmaceutical compositions.

For example, one pharmaceutical composition of such a kit may comprise a TAA binding antibody or binding fragment thereof such as a NY-ESO-1 binding antibody or binding fragment thereof while a second pharmaceutical composition may comprise or at least one agonistic activator of natural stimulants or at least co-stimulants of the immune system such as anti-CD40 agonistic antibodies or at least one antagonistic effector of natural inhibitors or at least co-inhibitors of the immune system such as anti-CTLA4 antagonistic antibodies.

In embodiments where the kit comprises a TAA binding antibody or binding fragment thereof and both at least one agonistic activator of natural stimulants or at least co-stimulants of the immune system such as anti-CD40 agonistic antibodies, and at least one antagonistic effector of natural inhibitors or at least co-inhibitors of the immune system such as anti-CTLA4 antagonistic antibodies, one pharmaceutical composition of such a kit may comprise a TAA binding antibody or binding fragment thereof such as a NY-ESO-1 binding antibody or binding fragment thereof, while a second pharmaceutical composition may comprise at least one agonistic activator of natural stimulants or at least co-stimulants of the immune system such as anti-CD40 agonistic antibodies and a third pharmaceutical composition may comprise at least one antagonistic effector of natural inhibitors or at least co-inhibitors of the immune system such as anti-CTLA4 antagonistic antibodies. In alternative thereof, the second pharmaceutical composition may comprise both at least one agonistic activator of natural stimulants or at least co-stimulants of the immune system such as anti-CD40 agonistic antibodies and at least one antagonistic effector of natural inhibitors or at least co-inhibitors of the immune system such as anti-CTLA4 antagonistic antibodies.

Such kits allow treatment of patients by subsequent and/or at least partially simultaneous administration of the various pharmaceutical preparations which form the kit and may thus enable a timely optimized treatment regimen of the above mentioned combinations.

The present invention also relates to a combination of at least one tumor associated antigen (TAA) binding antibody or binding fragment thereof and at least one compound capable of activating the immune system for use in treating a disease such as a hyper-proliferative disease. The TAA binding antibody or binding fragments thereof and the at least one compound capable of activating the immune system may be selected as described hereinafter.

As is described hereinafter, the combinations of active agents in accordance with the invention, i.e. TAA binding antibodies or fragments thereof and compounds which are capable of stimulating the immune system, may provide improved efficacy if patients are subjected to cytotoxic treatment prior to, simultaneous with or subsequent to administration of the aforementioned pharmaceutical compositions, kits or combinations comprising such active agents. It may be preferred that patients receive such cytotoxic treatment prior to or simultaneous with administration of the aforementioned pharmaceutical compositions, kits or combinations comprising such active agents.

If such cytotoxic treatment comprises administration of cytotoxic agents, such cytotoxic agents may be included in the pharmaceutical compositions or kits in accordance with the invention. One exemplary preferred representative of such cytotoxic agents is 5-fluoro uracil (5-FU).

In a third aspect, the pharmaceutical compositions and kits in accordance with the invention may be used to treat patients suffering or being suspected to be prone to hyper-proliferative diseases, such as cancer.

Preferably, the pharmaceutical compositions and kits in accordance with the invention may be used to treat patients suffering or being suspected to be prone to cancers which are characterized by the expression of TAAs such as cancers being characterized by the expression of CT-antigens.

If the TAA binding antibody or binding fragment thereof which is comprised within the pharmaceutical compositions and kits in accordance with the invention is a NY-ESO-1 binding antibody or binding fragment thereof, the treatment of cancers such as non-small cell lung cancer, melanoma, esophageal cancer, bladder cancer, hepatocellular cancer or prostate cancer may be preferred. If the TAA binding antibody or binding fragment thereof is e.g. a MAGE-3 binding antibody or binding fragment thereof, the treatment of cancers such as melanoma, non-small cell cancer or multiple myeloma may be preferred. If the TAA binding antibody or binding fragment thereof is e.g. a MAGE-1 binding antibody or binding fragment thereof, the treatment of cancers such as non-small cell lung cancer, melanoma, hepatocellular cancer, bladder cancer, head and neck cancer or esophageal cancer may be preferred.

The present invention thus also relates to a medicament for use in treating a patient wherein a pharmaceutical composition or a kit as described hereinafter is used. TAA binding antibodies or binding fragments may preferably be CT-antigen binding antibodies or binding fragments thereof and compounds capable of activating the immune response may preferably be selected from at least one natural stimulant or at least co-stimulant of the immune system, agonistic activator of natural stimulants or at least co-stimulants of the immune system or at least one antagonistic effector of natural inhibitors or at least co-inhibitors of the immune system as described hereinafter. In some embodiments, a combination of the NY-ESO-1 binding antibodies or binding fragments thereof as mentioned herein, anti-CD40 agonistic antibodies such as CP-870,893 and SGN-40 and/or anti-CTLA4 antagonistic antibodies such as Tremelimumab and Ipilimumab are envisaged.

Such medicaments may be used for patients who are subjected to cytotoxic treatment prior to, simultaneous with or subsequent to administration of such medicaments. In one embodiment the cytotoxic treatment may include chemotherapy.

Such medicaments may in particular be used for treatment of hyper-proliferative disease such as cancer.

The present disclosure also relates to the use of a pharmaceutical composition or a kit as described hereinafter in the manufacture of a medicament for treating a patient. TAA binding antibodies or binding fragments may preferably be CT-antigen binding antibodies or binding fragments thereof and compounds capable of activating the immune response may preferably be selected from at least one natural stimulant or at least co-stimulant of the immune system, agonistic activator of natural stimulants or at least co-stimulants of the immune system or at least one antagonistic effector of natural inhibitors or at least co-inhibitors of the immune system as described hereinafter. In some embodiments, a combination of the NY-ESO-1 binding antibodies or binding fragments thereof as mentioned herein, anti-CD40 agonistic antibodies such as CP-870,893 and SGN-40 and/or anti-CTLA4 antagonistic antibodies such as Tremelimumab and Ipilimumab are envisaged.

Such medicaments may be used for patients which are subjected to cytotoxic treatment prior to, simultaneous with or subsequent to administration of such medicaments. In one embodiment the cytotoxic treatment may include chemotherapy.

Such medicaments may in particular be used for treatment of hyper-proliferative disease such as cancer.

The present disclosure also relates to a method of treating a patient by administering a pharmaceutical composition or a kit as described hereinafter to the patient. TAA binding antibodies or binding fragments may preferably be CT-antigen binding antibodies or binding fragments thereof and compounds capable of activating the immune response may preferably be selected from at least one natural stimulant or at least co-stimulant of the immune system, agonistic activator of natural stimulants or at least co-stimulants of the immune system or at least one antagonistic effector of natural inhibitors or at least co-inhibitors of the immune system as described hereinafter. In some embodiments, a combination of the NY-ESO-1 binding antibodies or binding fragments thereof as mentioned herein, anti-CD40 agonistic antibodies such as CP-870,893 and SGN-40 and/or anti-CTLA4 antagonistic antibodies such as Tremelimumab and Ipilimumab are envisaged.

Such methods may be considered for patients which are subjected to cytotoxic treatment prior to, simultaneous with or subsequent to administration of such medicaments. In one embodiment the cytotoxic treatment may include chemotherapy.

Such methods may be considered for treatment of hyper-proliferative disease such as cancer.

The present disclosure also relates to diagnostic compositions comprising the pharmaceutical compositions and kits in accordance with the invention and to the use of the pharmaceutical compositions and kits in accordance with the disclosure as diagnostic tools. These diagnostic compositions and tools can be used to diagnose patients for e.g. cancers being characterized by an altered expression of TAAs such as NY-ESO-1 and immune-modulating factors such as CD40 and CTLA4. The present disclosure further relates to the diagnostic methods where the afore-mentioned diagnostic compositions and tools are used.

In another aspect the present invention also relates to the individual specific NY-ESO-1 binding antibodies and binding fragments thereof as they are disclosed in the context of the present invention.

These antibodies or binding fragments thereof include 12D7, 12D7*, 31E4, 30D6, 15B12, 22A1, 1H12, 10E1 and 1D4. These antibodies or binding fragments thereof further include binding antibodies or fragments thereof comprising a variable heavy chain and/or a variable light chain of the exemplary antibodies 12D7, 12D7*, 31E4, 30D6, 15B12, 22A1, 1H12, 10E1 or 1D4 or a variable heavy chain and/or a variable light chain having at least 80% sequence identity with the variable heavy chain and/or variable light chain of the exemplary antibodies 12D7, 12D7*, 31E4, 30D6, 15B12, 22A1, 1H12, 10E1 or 1D4.

These antibodies or binding fragments thereof further include binding antibodies or fragments thereof comprising the complementary determining regions (CDRs) of the exemplary antibodies 12D7, 12D7*, 31E4, 30D6, 15B12, 22A1, 1H12, 10E1 or 1D4 within their variable heavy chain and/or variable light chain. Such antibodies or binding fragments thereof may also comprise CDRs within their variable heavy chain and/or variable light chain having at least 80% sequence identity with the CDRs of the exemplary antibodies 12D7, 12D7*, 31E4, 30D6, 15B12, 22A1, 1H12, 10E1 or 1D4.

All of these specific individual NY-ESO-1 binding antibodies or fragments thereof have in common that they have either been directly obtained from patients which have received a NY-ESO-1 vaccination and which have shown a favorable clinical course of disease or that they have been derived from antibodies of such patients. They thus are monoclonal, human, patient-derived antibodies. Comparable monoclonal human antibodies may be isolated from patients which have developed CT antigen binding antibodies such as e.g. NY-ESO-1 binding antibodies or MAGE binding antibodies spontaneously, i.e. in the absence of vaccination with NY-ESO-1 or MAGE, during tumor development and have shown a favorable clinical course of disease.

The present invention further relates to nucleic acid molecules encoding for such antibodies, to nucleic acid molecules encoding for the variable light and/or heavy chains thereof and to nucleic acid molecules encoding for the CDR1, CDR2 and/or CDR3 of the variable light and/or heavy chains thereof.

The present invention further relates to vectors comprising such nucleic acid molecules and/or such vectors.

The present invention also relates to pharmaceutical compositions comprising such specific NY-ESO-1 binding antibodies or binding fragments thereof.

The present invention further relates to pharmaceutical compositions comprising such specific NY-ESO-1 binding antibodies or binding fragments thereof for use in treating hyper-proliferative disease, in particular tumors which express NY-ESO-1.

The present invention further relates to the use of such specific NY-ESO-1 binding antibodies or binding fragments thereof in the manufacture of a medicament for treating hyper-proliferative diseases, in particular tumors which express NY-ESO-1.

The present invention further relates to methods of treating hyper-proliferative diseases, in particular tumors which express NY-ESO-1 by administering to patients such specific NY-ESO-1 binding antibodies or binding fragments thereof.

The present disclosure further relates to a diagnostic composition comprising such specific NY-ESO-1 binding antibodies or binding fragments thereof for use in diagnosing hyper-proliferative diseases, in particular tumors which express NY-ESO-1.

The present disclosure further relates to the use of such specific NY-ESO-1 binding antibodies or binding fragments thereof in the manufacture of a diagnostic composition for diagnosing hyper-proliferative diseases, in particular tumors which express NY-ESO-1.

The present disclosure further relates to methods of diagnosing hyper-proliferative diseases, in particular tumors which express NY-ESO-1 by using such specific NY-ESO-1 binding antibodies or binding fragments thereof.

### FIGURE LEGENDS

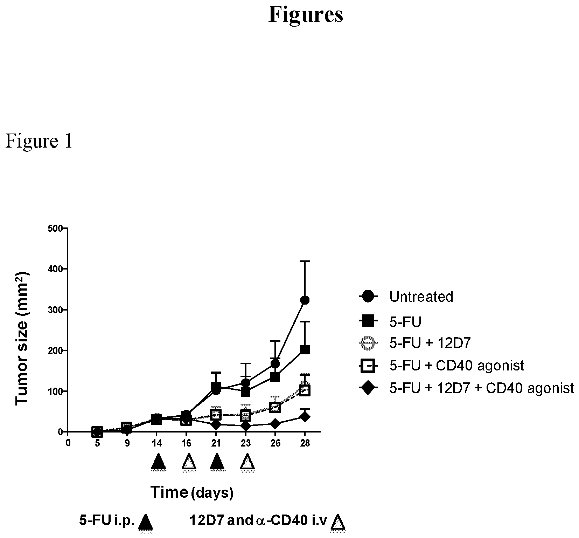

- Fig. 1:: Co-administration of CD40 agonistic antibody enhances reduction of tumor growth mediated by human monoclonal antibody anti-NY-ESO-1 plus chemotherapy.
Mice were inoculated with CT26/NY-ESO-1 mouse colon carcinoma cells on day 0. Mice were treated with 5-FU injected into the peritoneum on days 14 and 21. CD40 agonistic antibody was administered intravenously alone, or in combination with human monoclonal antibody anti-NY-ESO-1-12D7 on days 16 and 23. Tumor size (area) was measured on days 5; 9; 14; 16; 21; 23; 26 and 28. Treatment groups: Untreated, 5-FU alone (5-FU), 5-FU in combination with human monoclonal antibody anti-NY-ESO-1 12D7 (5FU+12D7), 5-FU with CD40 agonistic antibody (5-FU + CD40 agonist) and 5-FU with CD40 agonistic antibody and 12D7 (5-FU + 12D7+ CD40 agonist).

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denotes an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

The present invention is *inter alia* based on the experimental finding that mice with a syngeneic NY-ESO-1 positive colon tumor which were treated with 5-FU display infiltration of CD4⁺, CD8⁺ T-cells after administration of NY-ESO-1 binding antibody 12D1 and that this effect is more pronounced upon additional administration of anti-CD40 agonistic antibodies. As a consequence of these treatments, tumor size is reduced.

Without wanting to be held to this hypothesis, it is assumed that administration of TAA binding antibodies such as the CT-antigen binding antibody 12D7 triggers a immune response which from a therapeutic perspective (e.g. in terms of tumor destruction) is localized at the site of tumor. It seems that this type of localized immune response can be further augmented and/or prolonged by administration of compounds which are capable of activating the immune system such as the CD40 agonistic antibodies.

This combined approach of using very selective tumor targeting agents (i.e. the TAA binding antibodies) with what may be designated as broad band immuno-modulating agents (i.e. compounds capable of stimulating an immune response) and even nonspecific cytotoxic agents may provide several advantages that may significantly improve disease therapy.

Standard chemotherapy with compounds such as 5-FU, therapies focusing on general immuno-modulators e.g. via toll-7 or toll-9 receptor agonists, CD-40 receptor agonists, anti-CTLA-4 antagonistic antibodies and even more targeted therapies involving therapeutic antibodies directed against the EGF-Receptor or HER-2 receptor suffer from various side effects.

Chemotherapy with cytotoxic agents affects dividing cells in general. Immuno-modulators enhance other non-tumor directed immune reactions as well as adverse autoimmune reactions. Antibody addressed EGF-Receptors or HER-2 receptors are of functional relevance not only in tumor tissue but in other differentiated normal cells as well e.g. of the heart.

These properties lead to "off- target" (the target being the tumor) side effects which can e.g. limit the dosage and thus the effectiveness of these otherwise therapeutically extremely important therapeutic principles.

By the use of TAA binding antibodies and preferably of CT antigen binding antibodies which are monoclonal human patient-derived antibodies as described hereinafter, the therapeutically important effects of systemically active immune-modulators may be boosted as these activities seem to be more limited to the therapeutic areas of interest, namely the tumor tissue which is pre-selected through the TAA binding antibodies such as NY-ESO-1 binding antibodies. This assumed pre-selection of the therapeutic area of interest, namely the tumor tissue, by TAA binding antibodies and the focusing of the broad band activity of immune-modulating agents to these areas of therapeutic interest should limit off-target related adverse events at least to some extent. This should in turn allow e.g. using immuno-modulating agents such as anti-CD40 agonistic antibodies in higher concentrations than usual and to thus benefit to a greater extent from their therapeutic potential. One may also envisage more effective dosage regimens such as shortened intervals for subsequent administration of the pharmaceutically active agents.

Given that the initial immune response is selectively targeted by the TAA binding antibody to tumor tissue only, the additional augmentation seems to also preferentially only effect the tumor tissue only. Such a localized integrated tumor specific immune response may be particularly effective if chemotherapy with e.g. 5-FU makes the TAAs readily accessible for the TAA binding antibody.

Based on the above observations, it seems justified that the effects observed for the combination of 5-FU, 12D7 and anti-CD40 agonistic antibodies may also apply for other CT-antigen binding antibodies or TAA binding antibodies in general, for other activators of the immune system such as CD40L, anti-OX40 agonistic antibodies, anti-CD 137 agonistic antibodies, anti-CTLA4 antagonistic antibodies, anti PD-1 antagonistic antibodies or anti-CD25 antagonistic antibodies and for other cellular stress inducing therapies such as radiation.

The invention in one aspect is therefore directed to a pharmaceutical composition comprising at least one tumor associated antigen (TAA) binding antibody or binding fragment thereof and at least one compound capable of activating the immune system. The combination of such pharmaceutically active agents may also be comprised within a kit of pharmaceutical compositions as it is described hereinafter.

It should be understood that the term "kit" indicates that the disclosure considers the treatment of e.g. hyper-proliferative diseases as mentioned hereinafter by combinations of pharmaceutically active agents and these pharmaceutically active agents (e.g. an NY-ESO-1 binding antibody, an anti-CD40 agonistic antibody and/or an anti-CTLA4 antagonistic antibody) do not need to be combined with a single pharmaceutical dosage form. In fact, it may be advantageous to actually use e.g. an NY -ESO-1 binding antibody and an anti-CD40 agonistic antibody in the form of separately provided pharmaceutical dosage forms as this will allow accounting e.g. for different pharmacokinetic properties of these antibodies during treatment. The term "kit" therefore is also not to be understood as referring to e.g. necessarily simultaneously offering separate pharmaceutical dosage forms which comprise the pharmaceutically active agent even though such type of offering is not excluded. The term "kit" indicates that the invention focuses on a use of a combination of different pharmaceutically active agents during therapy and that this combination may e.g. be offered as separate single pharmaceutical dosage forms which can then be used in e.g. a method or use in accordance with the invention.

The present invention thus also relates to a combination of at least one tumor associated antigen (TAA) binding antibody or binding fragment thereof and at least one compound capable of activating the immune system for use in treating a disease such as a hyper-proliferative disease. The TAA binding antibody or binding fragments thereof and the at least one compound capable of activating the immune system may be selected as described hereinafter. The components of such combination may be used simultaneously or sequentially for treatment of e.g. hyper-proliferative diseases.

The term "Tumor Associated Antigen (TAA)" in its broadest sense relates to factors which are primarily, if not exclusively expressed in tumors and thus can act as potential immune-therapeutic targets for antibody-based therapy. The primary and preferably exclusive expression of TAAs in tumor tissue ensures that the therapeutic antibody mediated immune reaction will be localized to the tumor only so that the above-described adverse events and effects on pharmacokinetic behavior are observed at least not to the same extent as for therapeutic antibodies which target antigens that are expressed both in tumor and normal tissues.

It is to be understood that expression of such TAAs must be seen before the background of accessibility of such expressed TAAs to antibodies and/or accessibility of such expressed TAAs to the immune system.

Thus, expression of TAAs may occur on the DNA or RNA level in normal tissue which, however, does not translate into expression on the protein level. As a consequence such a TAA will not be expressed in normal tissues in an extent that would make it principally available for therapeutic antibodies as such antibodies are commonly understood to recognized antigens and/or epitopes involving stretches of amino acids.

Further, there may be tissues such as testis which are not functionally accessible to the immune system, e.g. in the sense that they do not show MHC expression and therefore cannot be targeted by T-cells, and which therefore are commonly considered to be immune privileged. Even if a TAA is expressed in such immune privileged normal tissue, an antibody binding to such a TAA would thus not trigger an immune response in such normal tissue. Again the immune response would be limited to the tumor tissue expressing the TAA.

A preferred group of TAAs are the so-called "cancer/testis antigens (CT-antigens)". This group has emerged as a unique class of TAAs which are expressed either in diverse tumors or normally in testis, i.e. an immune privileged tissue. An overview on the properties of CT-antigens including information on their genomic coding, function, tumor expression etc. can be found *inter alia* in Caballero et al., 2009, Cancer Science, 100(11), 2014-2021, the disclosure of which is incorporated by reference particularly with respect to the nature of CT antigens as well as the occurrence and distribution of specific CT antigens within different types of tumors (see e.g. Table 1 of Caballero et al., *vide supra*).

Detailed information about CT-antigens can be found in http://www.cta.lncc.br/). The information provided by this database, in particular with respect to gene families of CT-Antigens, specific family members, their chromosomal localization, CT identifiers and protein expression patterns in tumors are incorporated by reference.

TAA binding antibodies or binding fragments thereof in accordance with the disclosure bind to CT-antigens of Table 1.

**Table 1 - List of CT-Antigens**

| Gene family | CT-Antigen | CT-Identifier |
|---|---|---|
| MAGEA | MAGEA1 | CT1.1 |
| MAGEA | MAGEA2 | CT1.2 |
| MAGEA | MAGEA2B | |
| MAGEA | MAGEA3 | CT1.3 |
| MAGEA | MAGEA4 | CT1.4 |
| MAGEA | MAGEA5 | CT1.5 |
| MAGEA | MAGEA6 | CT1.6 |
| MAGEA | MAGEA8 | CT1.8 |
| MAGEA | MAGEA9 | CT1.9 |
| MAGEA | MAGEA9B/LOC728269 | |
| MAGEA | MAGEA10 | CT1.10 |
| MAGEA | MAGEA11 | CT1.11 |
| MAGEA | MAGEA 12 | CT1.12 |
| BAGE | BAGE | CT2.1 |
| BAGE | BAGE2 | CT2.2 |
| BAGE | BAGE3 | CT2.3 |
| BAGE | BAGE4 | CT2.4 |
| BAGE | BAGE5 | CT2.5 |
| MAGEB | MAGEB1 | CT3.1 |
| MAGEB | MAGEB2 | CT3.2 |
| MAGEB | MAGEB3 | CT3.5 |
| MAGEB | MAGEB4 | CT3.6 |
| MAGEB | MAGEB5 | CT3.3 |
| MAGEB | MAGEB6 | CT3.4 |
| GAGE | GAGE1 | CT4.1 |
| GAGE | GAGE2A | CT4.2 |
| GAGE | GAGE3 | CT4.3 |
| GAGE | GAGE4 | CT4.4 |
| GAGE | GAGE5 | CT4.5 |
| GAGE | GAGE6 | CT4.6 |
| GAGE | GAGE7 | CT4.7 |
| GAGE | GAGE12I | |
| GAGE | GAGE8 | CT4.8 |
| GAGE | GAGE12J | |
| GAGE | GAGE 13 | |
| GAGE | GAGE12B | |
| GAGE | GAGE12C | |
| GAGE | GAGE12D | |
| GAGE | GAGE12E | |
| GAGE | GAGE12F | |
| GAGE | GAGE12G | |
| GAGE | GAGE12H | |
| SSX | SSX1 | CT5.1 |
| SSX | SSX2 | CT5.2a |
| SSX | SSX2b | CT5.2b |
| SSX | SSX3 | CT5.3 |
| SSX | SSX4 | CT5.4 |
| SSX | SSX4B | |
| SSX | SSX5 | |
| SSX | SSX6 | |
| SSX | SSX7 | |
| SSX | SSX9 | |
| NY-ESO-1 | CTAG1B | CT6.1 |
| NY-ESO-1 | CTAG1A | |
| NY-ESO-1 | CTAG2 | CT6.2a |
| NY-ESO-1 | LAGE-1b | CT6.2b |
| MAGEC1 | MAGEC1 | CT7.1 |
| MAGEC1 | MAGEC3 | CT7.2 |
| ATAD2 | ATAD2 | 137 |
| SYCP1 | SYCP1 | CT8 |
| ZNF645 | ZNF645 | 138 |
| BRDT | BRDT | CT9 |
| MAGEC2 | MAGEC2 | CT10 |
| SPANX | SPANXA1 | CT11.1 |
| SPANX | SPANXA2 | |
| SPANX | SPANXB1 | CT11.2 |
| SPANX | SPANXB2 | |
| SPANX | SPANXC | CT11.3 |
| SPANX | SPANXD | CT11.4 |
| SPANX | SPANXE | |
| SPANX | SPANXN1 | CT11.6 |
| SPANX | SPANXN2 | CT811.7 |
| SPANX | SPANXN3 | CT11.8 |
| SPANX | SPANXN4 | CT11.9 |
| SPANX | SPANXN5 | CT11.10 |
| XAGE | XAGE 1 | CT12.1a |
| XAGE | XAGE1B | CT12.1b |
| XAGE | XAGE1C | CT12.1c |
| XAGE | XAGE1D | CT12.1d |
| XAGE | XAGE1E | |
| XAGE | XAGE2 | CT12.2 |
| XAGE | XAGE2B/CTD-2267G17.3 | |
| XAGE | XAGE3 | CT12.3a |
| XAGE | XAGE-3b | CT12.3b |
| XAGE | XAGE-4/RP11 - 167P23.2 | CT12.4 |
| XAGE | XAGE5 | CT12.5 |
| HAGE | DDX43 | CT13 |
| SAGE | SAGE1 | CT14 |
| ADAM2 | ADAM2 | CT15 |
| PAGE-5 | PAGE5 | CT16.1 |
| PAGE-5 | CT16.2 | CT16.2 |
| PAGE-5 | PAGE1 | CT16.3 |
| PAGE-5 | PAGE2 | CT16.4 |
| PAGE-5 | PAGE2B | CT16.5 |
| PAGE-5 | PAGE3 | CT16.6 |
| PAGE-5 | PAGE4 | CT16.7 |
| LIPI | LIPI | CT17 |
| NA88A pseudogene | VENTXP1 | CT18 |
| IL13RA | IL13RA2 | CT19 |
| TSP50 | TSP50 | CT20 |
| CTAGE-1 | CTAGE1 | CT21.1 |
| CTAGE-1 | CTAGE-2 | CT21.2 |
| CTAGE-1 | CTAGE5 | CT21.3 |
| SPA17 | SPA17 | CT22 |
| ACRBP | ACRBP | CT23 |
| CSAGE | CSAG1 | CT24.1 |
| CSAGE | CSAG2 | CT24.2 |
| CSAGE | CSAG3B | |
| MMA1 | DSCR8 | CT25.1a |
| MMA1 | MMAlb | CT25.1b |
| CAGE | DDX53 | CT26 |
| BORIS | CTCFL | CT27 |
| HOM-TES-85 | LUZP4 | CT28 |
| AF15q14 | CASC5 | CT29 |
| HCA661 | TFDP3 | CT30 |
| JARID1B | JARID1B | CT31 |
| LDHC | LDHC | CT32 |
| MORC | MORC1 | CT33 |
| SGY-1 | DKKL1 | CT34 |
| SPO11 | Spoil | CT35 |
| TPX1 | CRISP2 | CT36 |
| NY-SAR-35 | FMR1NB | CT37 |
| FTHL17 | FTHL17 | CT38 |
| NXF2 | NXF2 | CT39 |
| NXF2 | NXF2B | |
| TAF7L | TAF7L | CT40 |
| TDRD1 | TDRD1 | CT41.1 |
| TDRD1 | TDRD6 | CT41.2 |
| TEX15 | TEX15 | CT42 |
| FATE | FATE1 | CT43 |
| TPTE | TPTE | CT44 |
| CT45 | CT45A1 | CT45.1 |
| CT45 | CT45A2 | CT45.2 |
| CT45 | CT45A3 | CT45.3 |
| CT45 | CT45A4 | CT45.4 |
| CT45 | CT45A5 | CT45.5 |
| CT45 | CT45A6 | CT45.6 |
| HORMAD1 | HORMAD1 | CT46 |
| CT47 | CT47A1 | CT47.1 |
| CT47 | CT47A2 | CT47.2 |
| CT47 | CT47A3 | CT47.3 |
| CT47 | CT47A4 | CT47.4 |
| CT47 | CT47A5 | CT47.5 |
| CT47 | CT47A6 | CT47.6 |
| CT47 | CT47A7 | CT47.7 |
| CT47 | CT47A8 | CT47.8 |
| CT47 | CT47A9 | CT47.9 |
| CT47 | CT47A10 | CT47.10 |
| CT47 | CT47A11 | CT47.11 |
| CT47 | CT47B1 | CT47.13 |
| SLCO6A1 | SLCO6A1 | CT48 |
| TAG | TAG | CT49 |
| LEMD1 | LEMD1 | CT50 |
| HSPB9 | HSPB9 | CT51 |
| CCDC110 | CCDC110 | CT52 |
| ZNF165 | ZNF165 | CT53 |
| SPACA3 | SPACA3 | CT54 |
| CXorf48 | CXorf48 | CT55 |
| THEG | THEG | CT56 |
| ACTL8 | ACTL8 | CT57 |
| NLRP4 | NLRP4 | CT58 |
| COX6B2 | COX6B2 | CT59 |
| LOC348120 | LOC348120 | CT60 |
| CCDC33 | CCDC33 | CT61 |
| LOC196993 | LOC196993 | CT62 |
| PASD1 | PASD1 | CT63 |
| LOC647107 | LOC647107 | CT64 |
| TULP2 | TULP2 | CT65 |
| CT66 | CT66/AA884595 | CT66 |
| PRSS54 | PRSS54 | CT67 |
| RBM46 | RBM46 | CT68 |
| CT69 | CT69/BC040308 | CT69 |
| CT70 | CT70BI818097 | CT70 |
| SPINLW1 | SPINLW1 | CT71 |
| TSSK6 | TSSK6 | CT72 |
| ADAM29 | ADAM29 | CT73 |
| CCDC36 | CCDC36 | CT74 |
| LOC440934 | LOC440934 | CT75 |
| SYCE1 | SYCE1 | CT76 |
| CPXCR1 | CPXCR1 | CT77 |
| TSPY1 | TSPY3 | CT78 |
| TSPY1 | TSPY2 | |
| TSPY1 | LOC728137 | |
| TSPY1 | TSPY1D | |
| TSPY1 | TSPY1E | |
| TSPY1 | TSPY1F | |
| TSPY1 | TSPY1G | |
| TSPY1 | TSPY1H | |
| TSPY1 | TSPY1I | |
| TSGA10 | TSGA10 | CT79 |
| PIWIL2 | PIWIL2 | CT80 |
| ARMC3 | ARMC3 | CT81 |
| AKAP3 | AKAP3 | CT82 |
| Cxorf61 | Cxorf61 | CT83 |
| PBK | PBK | CT84 |
| C21orf99 | C21orf99 | CT85 |
| OIP5 | OIP5 | CT86 |
| CEP290 | CEP290 | CT87 |
| CABYR | CABYR | CT88 |
| SPAG9 | SPAG9 | CT89 |
| MPHOSPH1 | MPHOSPH1 | CT90 |
| ROPN1 | ROPN1 | CT91 |
| PLAC1 | PLAC1 | CT92 |
| CALR3 | CALR3 | CT93 |
| PRM | PRM2 | CT94.2 |
| PRM | PRM1 | CT94.1 |
| CAGE1 | CAGE1 | CT95 |
| CT96 | TTK | CT96 |
| LY6K | LY6K | CT97 |
| IMP-3 | IMP-3 | CT98 |
| AKAP4 | AKAP4 | CT99 |
| DPPA2 | DPPA2 | CT100 |
| KIAA0100/MLAA-22 | KIAA0100 | CT 101 |
| DCAF12 | DCAF12 | CT102 |
| SEMG1 | SEMG1 | CT103 |
| POTE | POTED | CT104.1 |
| POTE | POTEE | CT104.2 |
| POTE | POTEA | CT104.3 |
| POTE | POTEB | CT104.5 |
| POTE | POTEG | CT104.4 |
| POTE | POTEC | CT104.6 |
| POTE | POTEH | CT104.7 |
| GOLGAGL2FA | GOLGAGL2 FA | CT105 |
| NUF2/CDCA1 | CDCA1 | CT106 |
| RHOXF2/PEPP2 | PEPP2 | CT107 |
| OTOA | OTOA | CT108 |
| CCDC62 | CCDC62 | CT 109 |
| GPATCH2 | GPATCH2 | CT 110 |
| CEP55 | CEP55 | CT 111 |
| FAM46D | FAM46D | CT 112 |
| TEX14 | TEX14 | CT 113 |
| CTNNA2 | CTNNA2 | CT 114 |
| FAM133A | FAM133A | CT 115 |
| LYPD6B | LOC130576 | CT 116 |
| ANKRD45 | ANKRD45 | CT 117 |
| ELOVL4 | ELOVL4 | CT 118 |
| IGSF11 | IGSF11 | CT 119 |
| TMEFF | TMEFF1 | CT 120.1 |
| TMEFF | TMEFF2 | CT 120.2 |
| ARX | ARX | CT 121 |
| SPEF2 | SPEF2 | CT 122 |
| GPAT2 | GPAT2 | CT 123 |
| TMEM108 | TMEM108 | CT 124 |
| NOL4 | NOL4 | CT 125 |
| PTPN20A | PTPN20A | CT 126 |
| SPAG4 | SPAG4 | CT 127 |
| MAEL | MAEL | CT128 |
| RQCD1 | RQCD1 | CT 129 |
| PRAME | PRAME | CT130 |
| TEX 101 | TEX 101 | CT131 |
| SPATA19 | SPATA19 | CT132 |
| ODF1 | ODF1 | CT133 |
| ODF2 | ODF2 | CT134 |
| ODF3 | ODF3 | CT135 |
| ODF4 | ODF4 | CT136 |

Preferably, TAA binding antibodies or binding fragments thereof in accordance with the disclosure bind to CT-antigens of Table 2.

**Table 2 - List of preferred CT-antigens**

| Gene family | CT-Antigen | CT-Identifier |
|---|---|---|
| MAGEA | MAGEA1 | CT1.1 |
| MAGEA | MAGEA2 | CT1.2 |
| MAGEA | MAGEA3 | CT1.3 |
| MAGEA | MAGEA4 | CT1.4 |
| MAGEA | MAGEA5 | CT1.5 |
| MAGEA | MAGEA6 | CT1.6 |
| MAGEA | MAGEA10 | CT1.10 |
| BAGE | BAGE | CT2.1 |
| GAGE | GAGE1 | CT4.1 |
| SSX | SSX1 | CT5.1 |
| SSX | SSX2 | CT5.2a |
| SSX | SSX4 | CT5.4 |
| NY-ESO-1 | CTAG1B | CT6.1 |
| NY-ESO-1 | CTAG1A | |
| NY-ESO-1 | CTAG2 | CT6.2a |
| NY-ESO-1 | LAGE-1b | CT6.2b |
| MAGEC1 | MAGEC1 | CT7.1 |
| MAGEC1 | MAGEC3 | CT7.2 |
| MAGEC2 | MAGEC2 | CT10 |
| XAGE | XAGE 1 | CT12.1a |
| XAGE | XAGE2 | CT12.2 |
| CT47 | CT47A1 | CT47.1 |
| PRAME | PRAME | CT130 |

The term "CT-Antigen" is used interchangeably both for the gene family as well as for individual members of a gene family.

In a particular preferred embodiment, TAA binding antibodies or binding fragments thereof in accordance with the invention bind to CT-antigens of the NY-ESO-1 gene family.

In another embodiment, TAA binding antibodies or binding fragments thereof in accordance with the invention bind to CT-antigens of the MAGEA gene family or the MAGEC gene family.

It is to be understood that if in the following reference is made to TAA binding antibodies or binding fragments thereof or CT-antigen binding antibodies or binding fragments thereof, this always includes reference to NY-ESO-1 binding antibodies or fragments thereof and in particular the specific antibodies and their sequence homologues as they are mentioned herein such as 12D7, 12D7*, 31E4, 30D6, 15B12, 22A1, 1H12, 10E1 and 1D4.

If it is stated that an antibody or fragment thereof binds to a TAA such as CT-antigens, this means that the antibody or fragments thereof binds specifically to said antigen, i.e. binds the antigen with greater affinity than other antigens.

For example, an antibody or fragment is specific for its cognate antigen when the variable regions of the antibody or fragment recognize and bind the cognate antigen with a detectable preference distinguishing the antigen from other known polypeptides of similar but not identical sequence by virtue of measurable differences in binding affinity. It will be understood that specific antibodies and fragments may also interact with other proteins (for example, *S. aureus* protein A or other antibodies in ELISA techniques) through interactions with sequences outside the variable region of the antibodies, and in particular, in the constant region of the antibody or fragment. Screening assays to determine binding specificity of an antibody are well known and routinely practiced in the art. For a comprehensive discussion of such assays, see Harlow et al. (Eds), Antibodies A Laboratory Manual; Cold Spring Harbor Laboratory; Cold Spring Harbor, NY (1988), Chapter 6. As the TAAs contemplated in the context of the present invention are typically only expressed in tumor tissue or immune privileged tissue, specific binding antibodies or fragments thereof will preferably detectably bind (as judged by common assays) in tumor tissue to the TAAs only, but not to other polypeptides which are expressed both in tumor tissue and normal tissue.

Antibodies or binding fragments thereof, regardless of whether they are TAA binding antibodies or binding fragments thereof or e.g. the other antibodies described herein such as the anti-CD40 agonistic antibodies may have an equilibrium dissociation constant (K_{D}) for the binding of the antibody (or the binding fragment thereof) to its antigen in the low nanomolar to low picomolar or even in the subpicomolar range (avidity). Thus the K_{D} may be in the range of about 0.1*10⁻¹² to about 1*10⁻⁸, preferably in the range of about 0.1*10⁻¹² to about 0.1*10⁻⁷, more preferably in the range of about 0.1*10⁻¹² to about 10*10⁻⁹, even more preferably in the range of about 0.1*10⁻¹² to about 1*10⁻⁹. The most preferred KDs may be in the range of about 0.1*10⁻¹² to about 0.1*10⁻⁹, in the range of about 0.1*10⁻¹² to about 10*10⁻¹² or in the range of about 0.1*10⁻¹² to about 1*10⁻¹² such as about 0.9*10⁻¹², about 0.8*10⁻¹² about 0.7*10⁻¹², about 0.6*10⁻¹² or about 0.5*10⁻¹². The K_{D} is usually considered to be a measure for the affinity of an interaction between two molecules. Strictly speaking, affinity describes the strength of binding of a molecule to another molecule at a single site. However, an antibody usually has two binding sites for an antigen. The strength of this interaction is usually considered to be the avidity.

In the context of the present invention, the term "affinity" is used to describe both the strength of the interaction of e.g. a monovalent scFv to its antigen as well as the binding of a typical divalent antibody to its antigen.

K_{D} values and thus the affinity/avidity of the antibodies or binding fragments thereof can be determined making use of established approaches in the art.

The antibodies and binding fragments thereof as they are used in the context of the present invention, i.e. regardless of whether they are TAA binding antibodies or binding fragments thereof or e.g. the other antibodies described herein such as the anti-CD40 agonistic antibodies may be preferably monoclonal chimeric, humanized or human antibodies. These antibodies are preferably of the IgG class.

At least for the TAA binding antibodies or binding fragments thereof it can be preferred to use monoclonal human antibodies. Such antibodies are preferably "patient-derived".

A "patient-derived" human monoclonal antibody refers to an antibody which has been obtained from a patient suffering from a tumor and displaying a facorable clinical course of disease. Such favorable clinical course of disease may become apparent e.g. from quality of life, overall survival, improved time to progression and/or improved RECIST criteria. RECIST ("Response Evaluation Criteria In Solid Tumors") are e.g. use to determine whether a patient has shown a complete or at least partial response to treatment of such tumor. An explanation and overview of these criteria can be found *inter alia* at Eisenhauer et al., (2009) European Journal of Cancer, 228-247 or at http://www.eortc.be/recist/ and are incorporated by reference.

It is to be understood that a favorable clinical course of disease may be observed in patients which have been diagnosed with a tumor and which e.g. have received nonspecific chemotherapy and/or vaccination with an e.g. CT antigen. However, a patient which has shown a favorable clinical course of disease, may be eligible for isolation and identification of TAA binding antibodies even if the patient which has been diagnosed with a tumor, has not been e.g. vaccinated with a CT antigen.

The use of such patient-derived antibodies is assumed to provide for at least comparable efficacy even if they are administered to patients different from the ones from which they have been isolated. For example, the specific NY-ESO-1 binding antibodies or binding fragments thereof mentioned herein have been isolated from patients which were vaccinated with NY-ESO-1 and which showed at least a partial response towards such treatment. As demonstrated in the below experiments such antibodies are able to recruit CD4⁺, CD8⁺ cytotoxic T cells into xenografted tumors of mice.

As mentioned above, it can be preferred to use NY-ESO-1 binding antibodies or binding fragments thereof in the context of the present disclosure, for example in combination with anti-CD40 agonistic antibodies or agonistic binding fragments thereof and/or anti-CTLA4 antagonistic antibodies for treating hyper-proliferative disease such as cancers which express NY-ESO-1.

Preferably such NY-ESO-1 binding antibodies or binding fragments thereof are monoclonal humanized or human antibodies. In a further preferred embodiment such antibodies are monoclonal human patient-derived NY-ESO-1 binding antibodies or binding fragments thereof.

Some examples of NY-ESO-1 binding antibodies include 12D7, 12D7*, 31E4, 30D6, 15B12, 22A1, 1H12, 10E1 or 1D4.

The variable heavy chain of 12D7 is e.g. encoded by SEQ ID No. 1. The variable light chain of 12D7 is e.g. encoded by SEQ ID No. 2. The variable heavy chain of 12D7 thus has an amino acid sequence of SEQ ID No. 3. The variable light chain of 12D7 thus has an amino acid sequence of SEQ ID No. 4. As regards the variable heavy chain of 12D7, the CDR1 has an amino acid sequence of SEQ ID No. 5, the CDR2 has an amino acid sequence of SEQ ID No. 6 and the CDR3 has an amino acid sequence of SEQ ID No. 7. As regards the variable light chain of 12D7, the CDR1 has an amino acid sequence of SEQ ID No. 8, the CDR2 has an amino acid sequence of SEQ ID No. 9 and the CDR3 has an amino acid sequence of SEQ ID No. 10.

12D7* differs from 12D7 in the first 7 amino acids of the framework region 1 of the Ig-heavy- and Ig-light chains. Otherwise the amino acid sequences and in particular the CDRs are identical between 12D7* and 12D7. The DNA sequences differ since a codon optimization had been performed on 12D7* in order to optimize expression. The variable heavy chain of 12D7* is thus e.g. encoded by SEQ ID No. 11. The variable light chain of 12D7* is e.g. encoded by SEQ ID No. 12. The variable heavy chain of 12D7* thus has an amino acid sequence of SEQ ID No. 13. The variable light chain of 12D7* thus has an amino acid sequence of SEQ ID No. 14. As regards the variable heavy chain of 12D7*, the CDR1 has an amino acid sequence of SEQ ID No. 5, the CDR2 has an amino acid sequence of SEQ ID No. 6 and the CDR3 has an amino acid sequence of SEQ ID No. 7. As regards the variable light chain of 12D7*, the CDR1 has an amino acid sequence of SEQ ID No. 8, the CDR2 has an amino acid sequence of SEQ ID No. 9 and the CDR3 has an amino acid sequence of SEQ ID No. 10.

The variable heavy chain of 31E4 is e.g. encoded by SEQ ID No. 15. The variable light chain of 31E4 is e.g. encoded by SEQ ID No. 16. The variable heavy chain of 31E4 thus has an amino acid sequence of SEQ ID No. 17. The variable light chain of 31E4 thus has an amino acid sequence of SEQ ID No. 18. As regards the variable heavy chain of 31E4, the CDR1 has an amino acid sequence of SEQ ID No. 19, the CDR2 has an amino acid sequence of SEQ ID No. 20 and the CDR3 has an amino acid sequence of SEQ ID No. 21. As regards the variable light chain of 31E4, the CDR1 has an amino acid sequence of SEQ ID No. 22, the CDR2 has an amino acid sequence of SEQ ID No. 23 and the CDR3 has an amino acid sequence of SEQ ID No. 24.

The variable heavy chain of 30D6 is e.g. encoded by SEQ ID No. 25 or 26. The variable light chain of 30D6 is e.g. encoded by SEQ ID No. 27 or 28. The variable heavy chain of 30D6 thus has an amino acid sequence of SEQ ID No. 29. The variable light chain of 30D6 thus has an amino acid sequence of SEQ ID No. 30. As regards the variable heavy chain of 30D6, the CDR1 has an amino acid sequence of SEQ ID No. 31, the CDR2 has an amino acid sequence of SEQ ID No. 32 and the CDR3 has an amino acid sequence of SEQ ID No. 33. As regards the variable light chain of 30D6, the CDR1 has an amino acid sequence of SEQ ID No. 34, the CDR2 has an amino acid sequence of SEQ ID No. 35 and the CDR3 has an amino acid sequence of SEQ ID No. 36.

The variable heavy chain of 15B 12 is e.g. encoded by SEQ ID No. 37. The variable light chain of 15B 12 is e.g. encoded by SEQ ID No. 38. The variable heavy chain of 15B12 thus has an amino acid sequence of SEQ ID No. 39. The variable light chain of 15B12 thus has an amino acid sequence of SEQ ID No. 40. As regards the variable heavy chain of 15B12, the CDR1 has an amino acid sequence of SEQ ID No. 41, the CDR2 has an amino acid sequence of SEQ ID No. 42 and the CDR3 has an amino acid sequence of SEQ ID No. 43. As regards the variable light chain of 15B12, the CDR1 has an amino acid sequence of SEQ ID No. 44, the CDR2 has an amino acid sequence of SEQ ID No. 45 and the CDR3 has an amino acid sequence of SEQ ID No. 46.

The variable heavy chain of 22A1 is e.g. encoded by SEQ ID No. 47. The variable light chain of 22A1 is e.g. encoded by SEQ ID No. 48. The variable heavy chain of 22A1 thus has an amino acid sequence of SEQ ID No. 49. The variable light chain of 22A1 thus has an amino acid sequence of SEQ ID No. 50. As regards the variable heavy chain of 22A1, the CDR1 has an amino acid sequence of SEQ ID No. 51, the CDR2 has an amino acid sequence of SEQ ID No. 52 and the CDR3 has an amino acid sequence of SEQ ID No. 53. As regards the variable light chain of 22A1, the CDR1 has an amino acid sequence of SEQ ID No. 54, the CDR2 has an amino acid sequence of SEQ ID No. 55 and the CDR3 has an amino acid sequence of SEQ ID No. 56.

The variable heavy chain of 1H12 is e.g. encoded by SEQ ID No. 57. The variable light chain of 1H12 is e.g. encoded by SEQ ID No. 58. The variable heavy chain of 1H12 thus has an amino acid sequence of SEQ ID No. 59. The variable light chain of 1H12 thus has an amino acid sequence of SEQ ID No. 60. As regards the variable heavy chain of 1H12, the CDR1 has an amino acid sequence of SEQ ID No. 61, the CDR2 has an amino acid sequence of SEQ ID No. 62 and the CDR3 has an amino acid sequence of SEQ ID No. 63. As regards the variable light chain of 1H12, the CDR1 has an amino acid sequence of SEQ ID No. 64, the CDR2 has an amino acid sequence of SEQ ID No. 65 and the CDR3 has an amino acid sequence of SEQ ID No. 66.

The variable heavy chain of 10E1 is e.g. encoded by SEQ ID No. 67. The variable light chain of 10E1 is e.g. encoded by SEQ ID No. 68. The variable heavy chain of 10E1 thus has an amino acid sequence of SEQ ID No. 69. The variable light chain of 10E1 thus has an amino acid sequence of SEQ ID No. 70. As regards the variable heavy chain of 10E1, the CDR1 has an amino acid sequence of SEQ ID No. 71, the CDR2 has an amino acid sequence of SEQ ID No. 72 and the CDR3 has an amino acid sequence of SEQ ID No. 73. As regards the variable light chain of 10E1, the CDR1 has an amino acid sequence of SEQ ID No. 74, the CDR2 has an amino acid sequence of SEQ ID No. 75 and the CDR3 has an amino acid sequence of SEQ ID No. 76.

The variable heavy chain of 1D4 is e.g. encoded by SEQ ID No. 77. The variable light chain of 1D4 is e.g. encoded by SEQ ID No. 78. The variable heavy chain of 1D4 thus has an amino acid sequence of SEQ ID No. 79. The variable light chain of 1D4 thus has an amino acid sequence of SEQ ID No. 80. As regards the variable heavy chain of 1D4, the CDR1 has an amino acid sequence of SEQ ID No. 81, the CDR2 has an amino acid sequence of SEQ ID No. 82 and the CDR3 has an amino acid sequence of SEQ ID No. 83. As regards the variable light chain of 1D4, the CDR1 has an amino acid sequence of SEQ ID No. 84, the CDR2 has an amino acid sequence of SEQ ID No. 85 and the CDR3 has an amino acid sequence of SEQ ID No. 86.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 8, 22, 34, 44, 54, 64, 74, 84 or sequences at least 80% identical thereto, a CDR2 selected from SEQ ID Nos.: 9, 23, 35, 45, 55, 65, 75, 85 or sequences at least 80% identical thereto, and/or a CDR3 selected from SEQ ID Nos.: 10, 24, 36, 46, 56, 66, 76, 86 or sequences at least 80% identical thereto; and/or wherein
b) the heavy chain variable region comprises at least a CDR1 selected from SEQ ID Nos.: 5, 19, 31, 41, 51, 61, 71, 81 or sequences at least 80% identical thereto, a CDR2 selected from SEQ ID Nos.: 6, 20, 32, 42, 52, 62, 72, 82 or sequences at least 80% identical thereto, and/or a CDR3 selected from SEQ ID Nos.: 7, 21, 33, 43, 53, 63, 73, 83 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 8 or sequences at least 80% identical thereto, a CDR2 selected from SEQ ID Nos.: 9 or sequences at least 80% identical thereto, and/or a CDR3 selected from SEQ ID Nos.: 10 or sequences at least 80% identical thereto; and/or wherein
b) the heavy chain variable region comprises at least a CDR1 selected from SEQ ID Nos.: 5 or sequences at least 80% identical thereto, a CDR2 selected from SEQ ID Nos.: 6 or sequences at least 80% identical thereto, and/or a CDR3 selected from SEQ ID Nos.: 7 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 22 or sequences at least 80% identical thereto, a CDR2 selected from SEQ ID Nos.: 23 or sequences at least 80% identical thereto, and/or a CDR3 selected from SEQ ID Nos.: 24 or sequences at least 80% identical thereto; and/or wherein
b) the heavy chain variable region comprises at least a CDR1 selected from SEQ ID Nos.: 19 or sequences at least 80% identical thereto, a CDR2 selected from SEQ ID Nos.: 20 or sequences at least 80% identical thereto, and/or a CDR3 selected from SEQ ID Nos.: 21 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 34 or sequences at least 80% identical thereto, a CDR2 selected from SEQ ID Nos.: 35 or sequences at least 80% identical thereto, and/or a CDR3 selected from SEQ ID Nos.: 36 or sequences at least 80% identical thereto; and/or wherein
b) the heavy chain variable region comprises at least a CDR1 selected from SEQ ID Nos.: 31 or sequences at least 80% identical thereto, a CDR2 selected from SEQ ID Nos.: 32 or sequences at least 80% identical thereto, and/or a CDR3 selected from SEQ ID Nos.: 33 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 44 or sequences at least 80% identical thereto, a CDR2 selected from SEQ ID Nos.: 45 or sequences at least 80% identical thereto, and/or a CDR3 selected from SEQ ID Nos.: 46 or sequences at least 80% identical thereto; and/or wherein
b) the heavy chain variable region comprises at least a CDR1 selected from SEQ ID Nos.: 41 or sequences at least 80% identical thereto, a CDR2 selected from SEQ ID Nos.: 42 or sequences at least 80% identical thereto, and/or a CDR3 selected from SEQ ID Nos.: 43 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 54 or sequences at least 80% identical thereto, a CDR2 selected from SEQ ID Nos.: 55 or sequences at least 80% identical thereto, and/or a CDR3 selected from SEQ ID Nos.: 56 or sequences at least 80% identical thereto; and/or wherein
b) the heavy chain variable region comprises at least a CDR1 selected from SEQ ID Nos.: 51 or sequences at least 80% identical thereto, a CDR2 selected from SEQ ID Nos.: 52 or sequences at least 80% identical thereto, and/or a CDR3 selected from SEQ ID Nos.: 53 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 64 or sequences at least 80% identical thereto, a CDR2 selected from SEQ ID Nos.: 65 or sequences at least 80% identical thereto, and/or a CDR3 selected from SEQ ID Nos.: 66 or sequences at least 80% identical thereto; and/or wherein
b) the heavy chain variable region comprises at least a CDR1 selected from SEQ ID Nos.: 61 or sequences at least 80% identical thereto, a CDR2 selected from SEQ ID Nos.: 62 or sequences at least 80% identical thereto, and/or a CDR3 selected from SEQ ID Nos.: 63 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 74 or sequences at least 80% identical thereto, a CDR2 selected from SEQ ID Nos.: 75 or sequences at least 80% identical thereto, and/or a CDR3 selected from SEQ ID Nos.: 76 or sequences at least 80% identical thereto; and/or wherein
b) the heavy chain variable region comprises at least a CDR1 selected from SEQ ID Nos.: 71 or sequences at least 80% identical thereto, a CDR2 selected from SEQ ID Nos.: 72 or sequences at least 80% identical thereto, and/or a CDR3 selected from SEQ ID Nos.: 73 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 84 or sequences at least 80% identical thereto, a CDR2 selected from SEQ ID Nos.: 85 or sequences at least 80% identical thereto, and/or a CDR3 selected from SEQ ID Nos.: 86 or sequences at least 80% identical thereto; and/or wherein
b) the heavy chain variable region comprises at least a CDR1 selected from SEQ ID Nos.: 81 or sequences at least 80% identical thereto, a CDR2 selected from SEQ ID Nos.: 82 or sequences at least 80% identical thereto, and/or a CDR3 selected from SEQ ID Nos.: 83 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 8, 22, 34, 44, 54, 64, 74, 84 or sequences at least 80% identical thereto, a CDR2 selected from SEQ ID Nos.: 9, 23, 35, 45, 55, 65, 75, 85 or sequences at least 80% identical thereto, and a CDR3 selected from SEQ ID Nos.: 10, 24, 36, 46, 56, 66, 76, 86 or sequences at least 80% identical thereto; and/or wherein
b) the heavy chain variable region comprises at least a CDR1 selected from SEQ ID Nos.: 5, 19, 31, 41, 51, 61, 71, 81 or sequences at least 80% identical thereto, a CDR2 selected from SEQ ID Nos.: 6, 20, 32, 42, 52, 62, 72, 82 or sequences at least 80% identical thereto, and a CDR3 selected from SEQ ID Nos.: 7, 21, 33, 43, 53, 63, 73, 83 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 8 or sequences at least 80% identical thereto, a CDR2 selected from SEQ ID Nos.: 9 or sequences at least 80% identical thereto, and a CDR3 selected from SEQ ID Nos.: 10 or sequences at least 80% identical thereto; and/or wherein
b) the heavy chain variable region comprises at least a CDR1 selected from SEQ ID Nos.: 5 or sequences at least 80% identical thereto, a CDR2 selected from SEQ ID Nos.: 6 or sequences at least 80% identical thereto, and a CDR3 selected from SEQ ID Nos.: 7 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 22 or sequences at least 80% identical thereto, a CDR2 selected from SEQ ID Nos.: 23 or sequences at least 80% identical thereto, and a CDR3 selected from SEQ ID Nos.: 24 or sequences at least 80% identical thereto; and/or wherein
b) the heavy chain variable region comprises at least a CDR1 selected from SEQ ID Nos.: 19 or sequences at least 80% identical thereto, a CDR2 selected from SEQ ID Nos.: 20 or sequences at least 80% identical thereto, and a CDR3 selected from SEQ ID Nos.: 21 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 34 or sequences at least 80% identical thereto, a CDR2 selected from SEQ ID Nos.: 35 or sequences at least 80% identical thereto, and a CDR3 selected from SEQ ID Nos.: 36 or sequences at least 80% identical thereto; and/or wherein
b) the heavy chain variable region comprises at least a CDR1 selected from SEQ ID Nos.: 31 or sequences at least 80% identical thereto, a CDR2 selected from SEQ ID Nos.: 32 or sequences at least 80% identical thereto, and a CDR3 selected from SEQ ID Nos.: 33 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 44 or sequences at least 80% identical thereto, a CDR2 selected from SEQ ID Nos.: 45 or sequences at least 80% identical thereto, and a CDR3 selected from SEQ ID Nos.: 46 or sequences at least 80% identical thereto; and/or wherein
b) the heavy chain variable region comprises at least a CDR1 selected from SEQ ID Nos.: 41 or sequences at least 80% identical thereto, a CDR2 selected from SEQ ID Nos.: 42 or sequences at least 80% identical thereto, and a CDR3 selected from SEQ ID Nos.: 43 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 54 or sequences at least 80% identical thereto, a CDR2 selected from SEQ ID Nos.: 55 or sequences at least 80% identical thereto, and a CDR3 selected from SEQ ID Nos.: 56 or sequences at least 80% identical thereto; and/or wherein
b) the heavy chain variable region comprises at least a CDR1 selected from SEQ ID Nos.: 51 or sequences at least 80% identical thereto, a CDR2 selected from SEQ ID Nos.: 52 or sequences at least 80% identical thereto, and a CDR3 selected from SEQ ID Nos.: 53 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 64 or sequences at least 80% identical thereto, a CDR2 selected from SEQ ID Nos.: 65 or sequences at least 80% identical thereto, and a CDR3 selected from SEQ ID Nos.: 66 or sequences at least 80% identical thereto; and/or wherein
b) the heavy chain variable region comprises at least a CDR1 selected from SEQ ID Nos.: 61 or sequences at least 80% identical thereto, a CDR2 selected from SEQ ID Nos.: 62 or sequences at least 80% identical thereto, and a CDR3 selected from SEQ ID Nos.: 63 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 74 or sequences at least 80% identical thereto, a CDR2 selected from SEQ ID Nos.: 75 or sequences at least 80% identical thereto, and a CDR3 selected from SEQ ID Nos.: 76 or sequences at least 80% identical thereto; and/or wherein
b) the heavy chain variable region comprises at least a CDR1 selected from SEQ ID Nos.: 71 or sequences at least 80% identical thereto, a CDR2 selected from SEQ ID Nos.: 72 or sequences at least 80% identical thereto, and a CDR3 selected from SEQ ID Nos.: 73 or sequences at least 80% identical thereto.

Other examples of NY-ESO-1 binding antibodies or binding fragments thereof describe monoclonal antibodies or binding fragments thereof comprising a light chain variable region and/or a heavy chain variable region, wherein
a) the light chain variable region comprises at least a CDR1 selected from SEQ ID Nos.: 84 or sequences at least 80% identical thereto, a CDR2 selected from SEQ ID Nos.: 85 or sequences at least 80% identical thereto, and a CDR3 selected from SEQ ID Nos.: 86 or sequences at least 80% identical thereto; and/or wherein
b) the heavy chain variable region comprises at least a CDR1 selected from SEQ ID Nos.: 81 or sequences at least 80% identical thereto, a CDR2 selected from SEQ ID Nos.: 82 or sequences at least 80% identical thereto, and a CDR3 selected from SEQ ID Nos.: 83 or sequences at least 80% identical thereto.

In all these examples the sequence identity is at least about 85%, more preferably at least about 90%, even more preferably at least about 95% and most preferably at least about 98% or about 99%. Sequence identity may be determined over the whole length of the respective sequences.

The determination of percent identity between two sequences is preferably accomplished using the mathematical algorithm of Karlin and Altschul (1993) Proc. Natl. Acad. Sci USA 90: 5873-5877. Such an algorithm is incorporated into the BLASTn and BLASTp programs of Altschul et al. (1990) J. Mol. Biol. 215: 403-410 available at NCBI (http://www.ncbi.nlm.nih.gov/blast/Blast.cge).

The determination of percent identity is performed with the standard parameters of the BLASTn and BLASTp programs.

BLAST polynucleotide searches are performed with the BLASTn program.
For the general parameters, the "Max Target Sequences" box may be set to 100, the "Short queries" box may be ticked, the "Expect threshold" box may be set to 10 and the "Word Size" box may be set to 28. For the scoring parameters the "Match/mismatch Scores" may be set to 1,-2 and the "Gap Costs" box may be set to linear. For the Filters and Masking parameters, the "Low complexity regions" box may not be ticked, the "Species-specific repeats" box may not be ticked, the "Mask for lookup table only" box may be ticked, the "Mask lower case letters" box may not be ticked.

BLAST protein searches are performed with the BLASTp program. For the general parameters, the "Max Target Sequences" box may be set to 100, the "Short queries" box may be ticked, the "Expect threshold" box may be set to 10 and the "Word Size" box may be set to "3". For the scoring parameters the "Matrix" box may be set to "BLOSUM62", the "Gap Costs" Box may be set to "Existence: 11 Extension:1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked.

The above-mentioned CDRs of a light and heavy chain variable region are preferably embedded in the framework and constant region of a human-derived antibody, i.e. in the sequences as determined for antibodies obtained from human patients as described herein. Preferably these antibodies are of the IgG class.

However, the above-mentioned CDRs of a light and heavy chain variable region may also be embedded in human sequences of framework and constant regions derived from other human antibodies, particularly if such sequences have been shown to be effective in antibody dependent cell mediated cytotoxicity (ADCC). In this context, one may e.g. use the human constant and framework sequences of humanized therapeutic antibodies that have been successfully used for therapeutic applications. The above-mentioned CDRs of a light and heavy chain variable region are preferably incorporated into the framework and constant regions of such humanized antibodies of the human IgG class.

Further, the above-mentioned CDRs of a light and heavy chain variable region may be embedded in essentially human sequences for framework and constant regions. However, particularly the framework regions, but also the constant regions may comprise amino acids as they are e.g. typically found in mouse antibodies which are known to enhance antigen binding and/or e.g. ADCC (see e.g. European patent application EP 0 451 216). Preferably these antibodies are of the IgG class.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and/or a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 4 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 14 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 18 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 30 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 40 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 50 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 60 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 70 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 80 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 3 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 13 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 17 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 29 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 39 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 49 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 59 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 69 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID No.: 79 or sequences at least 80% identical thereto.

Other NY-ESO-1 binding antibodies and binding fragments relate to antibodies or binding fragments thereof comprising a light chain variable region comprising SEQ ID Nos.: 4, 14, 18, 30, 40, 50, 60, 70, 80 or sequences at least 80% identical thereto and a heavy chain variable region comprising SEQ ID Nos.: 3, 13, 17, 29, 39, 49, 59, 69, 79 or sequences at least 80% identical thereto.

Other NY-ESO-1 binding antibodies and binding fragments relate to antibodies or binding fragments thereof comprising a light chain variable region comprising SEQ ID No.: 4 or sequences at least 80% identical thereto and a heavy chain variable region comprising SEQ ID No.: 3 or sequences at least 80% identical thereto.

Other NY-ESO-1 binding antibodies and binding fragments relate to antibodies or binding fragments thereof comprising a light chain variable region comprising SEQ ID No.: 14 or sequences at least 80% identical thereto and a heavy chain variable region comprising SEQ ID No.: 13 or sequences at least 80% identical thereto.

Other NY-ESO-1 binding antibodies and binding fragments relate to antibodies or binding fragments thereof comprising a light chain variable region comprising SEQ ID No.: 18 or sequences at least 80% identical thereto and a heavy chain variable region comprising SEQ ID No.: 17 or sequences at least 80% identical thereto.

Other NY-ESO-1 binding antibodies and binding fragments relate to antibodies or binding fragments thereof comprising a light chain variable region comprising SEQ ID No.: 30 or sequences at least 80% identical thereto and a heavy chain variable region comprising SEQ ID No.: 29 or sequences at least 80% identical thereto.

Other NY-ESO-1 binding antibodies and binding fragments relate to antibodies or binding fragments thereof comprising a light chain variable region comprising SEQ ID No.: 40 or sequences at least 80% identical thereto and a heavy chain variable region comprising SEQ ID No.: 39 or sequences at least 80% identical thereto.

Other NY-ESO-1 binding antibodies and binding fragments relate to antibodies or binding fragments thereof comprising a light chain variable region comprising SEQ ID No.: 50 or sequences at least 80% identical thereto and a heavy chain variable region comprising SEQ ID No.: 49 or sequences at least 80% identical thereto.

Other NY-ESO-1 binding antibodies and binding fragments relate to antibodies or binding fragments thereof comprising a light chain variable region comprising SEQ ID No.: 60 or sequences at least 80% identical thereto and a heavy chain variable region comprising SEQ ID No.: 59 or sequences at least 80% identical thereto.

Other NY-ESO-1 binding antibodies and binding fragments relate to antibodies or binding fragments thereof comprising a light chain variable region comprising SEQ ID No.: 70 or sequences at least 80% identical thereto and a heavy chain variable region comprising SEQ ID No.: 69 or sequences at least 80% identical thereto.

Other NY-ESO-1 binding antibodies and binding fragments relate to antibodies or binding fragments thereof comprising a light chain variable region comprising SEQ ID No.: 80 or sequences at least 80% identical thereto and a heavy chain variable region comprising SEQ ID No.: 79 or sequences at least 80% identical thereto.

Preferably, in all these examples the sequence identity is at least about 85%, more preferably at least about 90%, even more preferably at least about 95% and most preferably at least about 98% or at least about 99%. Sequence identity is determined as described above. Sequence identity may be determined over the whole length of the respective sequence.

The above-mentioned light and heavy chain variable regions are preferably embedded in the constant regions of a human-derived antibody, i.e. in the sequences as determined for antibodies obtained from human patients as described herein. Preferably these antibodies are of the IgG class.

However, the above-mentioned light and heavy chain variable regions may also be embedded in human sequences of constant regions derived from other human antibodies, particularly if such sequences have been shown to be effective in ADCC. In this context, one may e.g. use the human constant sequences of humanized therapeutic antibodies that have been successfully used for therapeutic applications. The above-mentioned light and heavy chain variable regions are preferably incorporated into the constant regions of such humanized antibodies of the human IgG class.

Further, the above-mentioned light and heavy chain variable regions may be embedded in essentially human sequences for constant regions. However, the constant regions may comprise amino acids as they are e.g. typically found in mouse antibodies which are known to enhance ADCC. Preferably these antibodies are of the IgG class.

The disclosure also contemplates using NY-ESO-1 antibodies and binding fragments thereof binding substantially to the same epitope or parts of the same epitope as do the NY-ESO-1 binding antibodies and binding fragments as described above.

Further, the disclosure considers using NY-ESO-1 antibodies and binding fragments thereof competing with NY-ESO-1 binding antibodies and binding fragments thereof as described above.

Epitope mapping may be undertaken by producing different fragments of the TAA such as NY-ESO-1 and to then test these fragments for binding to antibodies or the binding fragments thereof. Binding may be measured using a Biacore®. One may also use commercially available peptide arrays such as PepSpot™ from JPT Peptide Technologies GmbH (Berlin, Germany), or proteomics-based mass spectrometry methods. Competition for binding to a particular antigen or epitope can be determined using assays known in the art. For example one may label an antibody in accordance with the invention and test for its binding to NY-ESO-1. Subsequently, one adds unlabeled 12D7 (or any other NY-ESO-1 binding antibody) and determines whether it affects binding of the labeled antibody, or binding of the labeled antibody is studied in presence or absence of various concentrations of such unlabeled NY-ESO-1 binding antibody. Such label could be radioactive or fluorescent or other kinds of detectable label.

Competition for binding to a particular antigen or epitope is determined by a reduction in binding to antigen or epitope of at least about 50%, or at least about 70%, or at least about 80%, or at least about 90%, or at least about 95%, or at least about 99% or about 100% for the antibody in accordance with the invention. Binding may be measured using Biacore® equipment, various fluorescence detection technologies (e.g. Fluorescence correlation spectroscopy, fluorescence cross-correlation, Fluorescence Lifetime measurements etc.) or various types of radioimmunoassays or other assays used to follow antibody binding to a target molecule.

As mentioned above, the present invention considers TAA binding antibodies or binding fragments thereof. A full-length antibody includes a constant domain and a variable domain. The constant region need not be present in an antigen binding fragment of an antibody.

Binding fragments may thus include portions of an intact full length antibody, such as an antigen binding or variable region of the complete antibody. Examples of antibody fragments include Fab, F(ab')₂, Id and Fv fragments; diabodies; linear antibodies; single-chain antibody molecules (e.g., scFv); multispecific antibody fragments such as bispecific, trispecific, and multispecific antibodies (e.g., diabodies, triabodies, tetrabodies); minibodies; chelating recombinant antibodies; tribodies or bibodies; intrabodies; nanobodies; small modular immunopharmaceuticals (SMIP), binding-domain immunoglobulin fusion proteins; camelized antibodies; VHH containing antibodies; and any other polypeptides formed from antibody fragments. The skilled person is aware that the antigen-binding function of an antibody can be performed by fragments of a full-length antibody.

A Fab fragment consists of the VL, VH, CL and CH1 domains. An F(ab')² fragment comprises two Fab fragments linked by a disulfide bridge at the hinge region. An Fd is the VH and CH1 domains of a single arm of an antibody. An Fv fragment is the VL and VH domains of a single arm of an antibody.

Binding fragments also encompass monovalent or multivalent, or monomeric or multimeric (e.g. tetrameric), CDR-derived binding domains.

The TAA binding antibodies and binding fragments thereof may also encompass variants of the exemplary antibodies, binding fragments and sequences disclosed herein. Variants include peptides and polypeptides comprising one or more amino acid sequence substitutions, deletions, and/or additions that have the same or substantially the same affinity and specificity of epitope binding as one or more of the exemplary antibodies, fragments and sequences disclosed herein. Thus, variants include peptides and polypeptides comprising one or more amino acid sequence substitutions, deletions, and/or additions to the exemplary antibodies, fragments and sequences disclosed herein where such substitutions, deletions and/or additions do not cause substantial changes in affinity and specificity of epitope binding. For example, a variant of an antibody or fragment may result from one or more changes to an antibody or fragment comprising one or more of amino acid sequence of SEQ ID NOS: 3, 4 etc. or where the changed antibody or fragment has the same or substantially the same affinity and specificity of epitope binding as the starting sequence.

As mentioned, TAA binding antibodies and binding fragments thereof such as e.g. the aforementioned CT-antigen binding antibodies and binding fragments thereof including the specifically mentioned NY-Eso1 binding antibodies and fragments thereof are used in combination with compound capable of activating the immune system to augment and/or prolong the local immune response which has been triggered by the TAA binding antibody or binding fragment thereof.

The term "compound capable of activating the immune system" refers to a pharmaceutically acceptable compound which is capable of prolonging and/or augmenting an initial immune response which has been triggered by a TAA binding antibody or binding fragment thereof.

Such compounds can include compounds which are known to stimulate or at least co-stimulate a humoral or cellular immune response even if no a TAA binding antibody or binding fragment thereof has been administered prior to, simultaneous with or after administration of such compounds.

Preferably, the term "compound capable of activating the immune system" thus refers to a pharmaceutically acceptable compound which stimulates or at least co-stimulates e.g. maturation of Antigen Presenting Cells (APC) including e.g. dendritic cells, macrophages, neutrophils and eosinophils, T-cell activation, T-cell proliferation including e.g. CD4⁺ helper T-cell and/or CD8⁺ cytotoxic T-cell proliferation, expansion of T-cells, maintenance of memory T-cells and/or proliferation of NK cells. It is to be understood that for the purposes of the present Invention TAA binding antibodies or binding fragments thereof such as CT-antigen binding antibodies or binding fragments thereof are not considered as representatives of "compounds capable of activating the immune system".

The afore-mentioned "compounds capable of activating the immune system" may exert their activating function on the immune system through different mechanisms.

For example, "compounds capable of activating the immune system" may comprise natural components of the immune system which are known to be involved in the stimulation or at least co-stimulation of the aforementioned activities such as e.g. maturation of Antigen Presenting Cells (APC) including e.g. dendritic cells, macrophages, neutrophils or eosinophils, T-cell activation, T-cell proliferation including e.g. CD4⁺ helper T-cell and/or CD8⁺ cytotoxic T-cell proliferation, expansion of T-cells, maintenance of memory T-cells and/or proliferation of NK cells. Such natural components of the immune system which according to the disclosure are "compounds capable of activating the immune system" include CD40, CD40 Ligand (CD40L), CD80, CD80 Ligand, C86 and CD86 Ligand, DR5, B7, OX40, CD137, cytokines such as IL-2, IL-6, IL-8, IL-10, IL-12, TNF-a, MIP-1a, and others. These components form a subgroup of "compounds capable of activating the immune system" and may be designated as "natural stimulants or at least co-stimulants of the immune system". A preferred representative of this subgroup is CD40L.

"Compounds capable of activating the immune system" may, however, also comprise compounds which do not constitute natural components of the immune system but which induce and/or increase the activity of the afore-mentioned natural components of the immune system, i.e. have an agonistic effect on "natural stimulants or at least co-stimulants of the immune system". This subgroup of "compounds capable of activating the immune system" may be designated as "agonistic activators of natural stimulants or at least co-stimulants of the immune system". Embodiments of this latter subgroup comprise anti-CD40 agonistic antibodies such as CP-870,893, SGN-40, FGK45.5 or a humanized form thereof, anti-OX40 agonistic antibodies such as OX86, anti-CD 137 agonistic antibodies such as BMS-663513 and others. Information on such factors and antibodies can be taken *inter alia* from Weiner et al., (2010), Nature Reviews, 10, 317-327, Fonsatti et al., (2010), Seminars in Oncology, 37(5), 517-523 or Vonderheide (2007), Molecular Pathways, 13(4), 1083-1088.

Other "compounds capable of activating the immune system" includes compounds which release an inhibitory effect of natural components of the immune system on the aforementioned activities such as e.g. maturation of Antigen Presenting Cells (APC) including e.g. of dendritic cells, macrophages, neutrophils or eosinophils, T-cell activation, T-cell proliferation including e.g. CD4⁺ helper T-cell and/or CD8⁺ cytotoxic T-cell proliferation, expansion of T-cells, maintenance of memory T-cells and/or proliferation of NK cells. Examples of such natural components of the immune system which have an inhibitory or at least co-inhibitory effect on the afore-mentioned activities include e.g. CTLA4, CD25 PD-1 or sMICA. This further subgroup of "compounds capable of activating the immune system" may be designated as "antagonistic effectors of natural inhibitors or at least co-inhibitors of the immune system". Examples of "antagonistic effectors of natural inhibitors or at least co-inhibitors of the immune system" include anti-CTLA4 antagonistic antibodies such as Tremelimumab and Ipilimumab, anti-CD25 antagonistic antibodies such as Daclizumab and anti-PD1 antagonistic antibodies such as CT-011. Information on such factors and antibodies can be taken *inter alia* from Weber, (2008), The Oncologist, 13(suppl 4), 16-25 or Fonsatti et al., (2010), Seminars in Oncology, 37(5), 517-523.

In an embodiment of the disclosure "compounds capable of activating the immune system" are selected from CD40L, anti-CD40 agonistic antibodies including CP-870,893, and SGN-40 and anti-CTLA4 antagonistic antibodies including Tremelimumab and Ipilimumab.

It is to be understood that if antibodies such as anti-CD40 agonistic antibodies including CP-870,893, and SGN-40 or anti-CTLA4 antagonistic antibodies including Tremelimumab and Ipilimumab are used as compounds capable of activating the immune system, they may be used as binding fragments of the respective antibody.

Other "compounds capable of activating the immune system" include compounds which are known to act on the innate immune system such as activators of Toll-like receptors including Toll-like receptors, 2, 3, 4, 5, 7, 8, and 9. Such compounds include bacterial lipo protein, LPS, double-stranded RNA, poly I:C (polyinosinic polycytidylic acid), bacterial flagellin resiquimod (R848) and CpG-ODN.

As mentioned above, the TAA binding antibodies or binding fragments thereof may be combined with compounds capable of activating the immune system in different fashions.

Thus, a TAA binding antibody may be combined with natural stimulants or at least co-stimulants of the immune system, agonistic activators of natural stimulants or at least co-stimulants of the immune system or with antagonistic effectors of natural inhibitors or at least co-inhibitors of the immune system.

A specific example would be the combination of an NY-ESO-1 binding antibody as disclosed herein (such as 12D7) with anti-CD40 agonistic antibodies such as CP-870,893 or SGN-40, anti-OX40 agonistic antibodies such as OX86 and/or anti-CD 137 agonistic antibodies such as BMS-663513.

Another specific example would be the combination of an NY-ESO-1 binding antibody as disclosed herein (such as 12D7) with anti-CTLA4 antagonistic antibodies such as Tremelimumab or Ipilimumab and/or anti-CD25 antagonistic antibodies such as Daclizumab.

However, a TAA binding antibody may also be combined with e.g. (i) natural stimulants or at least co-stimulants of the immune system or agonistic activators of natural stimulants or at least co-stimulants of the immune system and (ii) with antagonistic effectors of natural inhibitors or at least co-inhibitors of the immune system.

A specific example would be the combination of an NY-ESO-1 binding antibody as disclosed herein (such as 12D7) with anti-CD40 agonistic antibodies such as CP-870,893 or SGN-40 and with anti-CTLA4 antagonistic antibodies such as Tremelimumab or Ipilimumab.

Other examples may further include OX86, BMS-663513, CT-011 and/or Daclizumab.

Further compounds which are known to act on the innate immune system such as activators of Toll-like receptors,2, 3, 4, 5, 7, 8, and 9 may be included.

A preferred embodiment comprises a combination of an NY-ESO-1 binding antibody as disclosed herein (such as 12D7) with anti-CD40 agonistic antibodies such as CP-870,893 or SGN-40 as the sole pharmaceutically active agents.

Another embodiment comprises a combination of an NY-ESO-1 binding antibody as disclosed herein (such as 12D7) with anti-CTLA4 antagonistic antibodies such as Tremelimumab or Ipilimumab as the sole pharmaceutically active agents.

Yet another embodiment comprises a combination of an NY-ESO-1 binding antibody as disclosed herein (such as 12D7) with anti-CD40 agonistic antibodies such as CP-870,893 or SGN-40 and with anti-CTLA4 antagonistic antibodies such as Tremelimumab or Ipilimumab as the sole pharmaceutically active agents.

It has been mentioned above that the different pharmaceutically active principles such as the TAA binding antibody or binding fragment thereof, natural stimulants or at least co-stimulants of the immune system, agonistic activators of natural stimulants or at least co-stimulants of the immune system or antagonistic effectors of natural inhibitors or at least co-inhibitors of the immune system may be combined within multi-specific antibodies such as bi-specific antibodies or binding fragments thereof. This will be illustrated for the specific example of a NY-ESO-1 binding antibody and an anti-CD40 agonistic or an anti-CTLA4 antagonistic antibody or binding fragment thereof. However, it will be understood that this principle can be extended to other compounds capable of activating the immune system as well.

Thus, a portion of a NY-ESO-1 binding antibody or binding fragment thereof and (i) a portion of an anti-CD40 agonistic antibody or binding fragment thereof or (ii) a portion of an anti-CTLA4 antagonistic antibody or binding fragment thereof may be combined in a bi-specific antibody.

Such bispecific antibodies or fragments can be of several configurations. For example, bispecific antibodies may resemble single antibodies (or antibody fragments) but have two different antigen binding sites (variable regions). Bispecific antibodies can be produced by chemical techniques (Kranz et al. (1981), Proc. Natl. Acad. Sci. USA, 78: 5807) or by recombinant DNA techniques. Bispecific antibodies can have binding specificities for at least two different epitopes, at least one of which is an epitope of the tumor associated antigen for which the antibody has been identified. The antibodies and binding fragments can also be heteroantibodies. Heteroantibodies are two or more antibodies, or antibody binding fragments (Fab) linked together, each antibody or fragment having a different specificity.

The use of such bispecific antibodies can have the advantage that the augmentation and/or prolongation of the initial localized immune response which is assumed to be triggered by the TAA binding antibody is confined to the tumor as precisely as possible.

This concept can, of course be extended to tri-specific antibodies which would comprise e.g. a portion of a NY-ESO-1 binding antibody or binding fragment thereof, a portion of an anti-CD40 agonistic antibody or binding fragment thereof and a portion of an anti-CTLA4 antagonistic antibody or binding fragment thereof.

As has been set out before, the afore-mentioned combinations may be provided in the form of a single pharmaceutical composition which would be the case e.g. for a bispecific antibody or they may be provided as a kit of pharmaceutical compositions.

Where a kit is contemplated, it may comprise the pharmaceutically active agents in separate pharmaceutical compositions in different combinations. This will again be illustrated for the specific example of a cytotoxic agent, a NY-ESO-1 binding antibody, an anti-CD40 agonistic antibody and an anti-CTLA4 antagonistic antibody. However, it will be understood that this principle can be adapted accordingly to other combinations.

In the aforementioned example, the kit may consist of two pharmaceutical compositions, the first pharmaceutical composition comprising the cytotoxic agent and the second pharmaceutical composition comprising a NY-ESO-1 binding antibody and an anti-CD40 agonistic antibody. This kit would allow to first treating a patient with chemotherapy which is assumed to make (in this case) the NY-ESO-1 antigen more readily accessible to the NY-ESO-1 binding antibody. However, the subsequent administration of the second pharmaceutical composition then ensures simultaneous delivery of both the NY-ESO-1 binding antibody and the anti-CD40 agonistic antibody. This will allow the anti-CD40 agonistic antibody to display its activity as soon as the NY-ESO-1 binding antibody has triggered a localized immune response.

In another example, the kit may consist of three pharmaceutical compositions, the first pharmaceutical composition comprising the cytotoxic agent, the second pharmaceutical composition comprising a NY-ESO-1 binding antibody and the third pharmaceutical composition comprising an anti-CTLA4 antagonistic antibody. This kit would allow to first treating a patient with chemotherapy which is assumed to make (in this case) the NY-ESO-1 antigen more readily accessible to the NY-ESO-1 binding antibody. The second and third pharmaceutical compositions could then be administered separately from each other to first trigger a localized immune response by the TAA binding antibody and to allow sufficient time for development of such an immune response before the anti-CTLA4 antagonistic antibody can fully exert its function. However, the anti-CTLA4 antibodies may also help to de-repress already existing NY-ESO-1 specific T-cells. These cells could be further activated by the subsequent administration of NY-ESO-1 specific antibodies which would further strengthen the NY-ESO-1 binding antibody mediated antigen presentation. For such case the third pharmaceutical composition may be administered before or at least concomitantly with the second pharmaceutical composition.

Such kits could thus be used to e.g. account for the different pharmacokinetic properties of the e.g. respective antibodies by a fine-tuned timely administration.

It has been mentioned above that the efficacy of the aforementioned combinations may be enhanced if the patients receiving such combinations are subjected to a cytotoxic treatment.

The term "cytotoxic treatment" includes chemotherapy, radiation therapy, surgery, hyperthermia and the like. Chemotherapy may include administration of cytotoxic agents such as taxanes including docetaxel and paclitaxel, anthracyclines, cisplatin, carboplatin, 5-fluoro-uracil, gemcitabine, capecitabin, navelbine or zoledronate.

Where chemotherapy and particularly the aforementioned cytotoxic agents are used as cytotoxic treatment, these agents may be included in the pharmaceutical compositions and kits as contemplated above. Preferably, 5-FU may be included.

The combinations of pharmaceutically active agents which may take the form of pharmaceutical compositions or kits as contemplated herein can be used as medicaments for use in treating patients suffering from hyper-proliferative diseases.

The combinations of pharmaceutically active agents which may take the form of pharmaceutical compositions or kits as contemplated herein can be also used in the manufacture of medicaments for treating patients suffering from hyper-proliferative diseases.

Further the combinations of pharmaceutically active agents which may take the form of pharmaceutical compositions or kits as contemplated herein can be administered in methods of treating patients suffering from hyper-proliferative diseases.

The term "hyper-proliferative disease" refers to diseases which are commonly designated as cancer or tumors.

Unless stated otherwise, the terms "cancer" and "tumor" are used interchangeably herein. These terms in particular relate but are not limited to cancer and tumors selected from the group comprising basal cell carcinoma; bladder cancer; bone cancer such as osteosarcoma; central nervous system tumors such as cerebellar astrocytoma, cerebral astrocytoma/malignant glioma, craniopharyngioma, ependymoblastoma, ependymoma, medulloblastoma, medulloepithelioma, pineal parenchymal tumors of intermediate differentiation, primitive neuroectodermal tumors, pineoblastoma and spinal cord tumors; Burkitt's lymphoma; breast cancer; cervical cancer; chronic myelogenous leukemia; colon cancer; rectal cancer; colorectal cancer; esophageal cancer; Ewing family of tumors; extrahepatic bile duct cancer; gallbladder cancer; gastrointestinal stromal tumor (GIST); glioma; head and neck cancer; islet cell tumors; Kaposi sarcoma; leukemia; liver cancer; lymphoma; Hodgkin's lymphoma; non-Hodgkin's lymphoma; T-cell lymphoma; mesothelioma; multiple myeloma/plasma cell neoplasm; myeloid leukemia; multiple myeloma; nasopharyngeal cancer; neuroblastoma; small cell lung cancer; non-small cell lung cancer; oropharyngeal cancer; osteosarcoma ; ovarian cancer; pancreatic cancer; parathyroid cancer; penile cancer; pharyngeal cancer; phaeochromocytoma; pituitary tumor; prostate cancer; renal cell cancer; respiratory tract carcinoma; retinoblastoma; skin cancer (melanoma); small intestine cancer; soft tissue sarcoma; squamous cell carcinoma; squamous neck cancer; stomach (gastric) cancer; testicular cancer; throat cancer; thyroid cancer; transitional cell cancer of the renal pelvis and ureter; urethral cancer; uterine cancer; vaginal cancer; vulvar cancer and Wilms tumor.

The cancers for which treatment is considered in the context of the present invention are typically those types of tumors which express at least to some degree the TAAs which are recognized by the TAA binding antibodies or binding fragments thereof.

Such cancers will preferably at least to some extent express CT-antigens such as NY-ESO-1 or MAGEA.

The efficacy of the combinations of pharmaceutically active agents which may take the form of pharmaceutical compositions or kits as contemplated herein, in the aforementioned uses and methods for treating specific types of the aforementioned cancers may to some extent depend on which TAA binding antibody or binding fragment is used.

If for example a NY-ESO-1 binding antibody is used as part of a pharmaceutical composition or kit in accordance with the invention, treatment of non-small cell lung cancer, melanoma, esophageal cancer, bladder cancer, hepatocellular cancer or prostate cancer may be particularly effective. However, treatment of ovary cancer, breast cancer (triple negative breast cancer and others), cervical cancer, multiple myeloma, colorectal cancer, esophageal cancer or head and neck cancer may also be envisaged.

If a MAGE-A1 binding antibody is used as part of a pharmaceutical composition or kit in accordance with the disclosure, treatment of non-small cell lung cancer, melanoma, hepatocellular cancer, bladder cancer, head and neck cancer or esophageal cancer may be particularly effective. However, treatment of pancreas cancer, neuroblastoma, sarcoma, ovary cancer, colorectal cancer, prostate cancer or breast cancer may also be envisaged.

If a MAGE-A2 binding antibody is used as part of a pharmaceutical composition or kit in accordance with the disclosure, treatment of melanoma may be particularly effective.

If a MAGE-A3 binding antibody is used as part of a pharmaceutical composition or kit in accordance with the disclosure, treatment of melanoma, breast cancer, ovarian cancer, non-small cell lung cancer, multiple myeloma and/or pancreatic cancer may be particularly effective.

If a MAGE-A4 binding antibody is used as part of a pharmaceutical composition or kit in accordance with the disclosure, treatment of melanoma, non-small cell cancer, multiple myeloma and/or serous ovarian carcinoma may be particularly effective.

If a MAGE-A10 binding antibody is used as part of a pharmaceutical composition or kit in accordance with the disclosure, treatment of non-small cell cancer may be particularly effective.

If a MAGE-C1 binding antibody is used as part of a pharmaceutical composition or kit in accordance with the disclosure, treatment of non-small cell cancer, hepatocellular carcinoma and/or multiple myeloma may be particularly effective.

The efficacy and/or selectivity of pharmaceutical compositions or kits in accordance with the disclosure towards certain cancers may be increased if different TAA binding antibodies or binding fragments which bind to e.g. different CT antigens are present are combined. Thus, the above-mentioned pharmaceutical compositions or kits may comprise combinations of NY-ESO-1, MAGE-A1, MAGE-A2, MAGE-A3, MAGE-A4, MAGE-10 and MAGE-C1 binding antibodies or binding fragments thereof with e.g. anti CD40 agonistic or binding fragments thereof and/or anti-CTLA4 antagonistic antibodies or binding fragments thereof.

As already mentioned, in another aspect the present disclosure also relates to the afore-mentioned individual specific NY-ESO-1 binding antibodies and binding fragments thereof as they are disclosed in the context of the present disclosure. The disclosure thus is also directed to these antibodies as such even if they are not in combination with e.g. compounds capable of activating an immune response.

These antibodies or binding fragments thereof include 12D7, 12D7*, 31E4, 30D6, 15B12, 22A1, 1H12, 10E1 and 1D4.

These antibodies or binding fragments thereof further include binding antibodies or fragments thereof comprising a variable heavy chain and/or a variable light chain of the exemplary antibodies 12D7, 12D7*, 31E4, 30D6, 15B12, 22A1, 1H12, 10E1 or 1D4 or a variable heavy chain and/or a variable light chain having at least 80% sequence identity with the variable heavy chain and/or variable light chain of the exemplary antibodies 12D7, 12D7*, 31E4, 30D6, 15B12, 22A1, 1H12, 10E1 or 1D4.

These antibodies or binding fragments thereof further include binding antibodies or fragments thereof comprising the complementary determining regions (CDRs) of the exemplary antibodies 12D7, 12D7*, 31E4, 30D6, 15B12, 22A1, 1H12, 10E1 or 1D4 within their variable heavy chain and/or variable light chain. Such antibodies or binding fragments thereof may also comprise CDRs within their variable heavy chain and/or variable light chain having at least 80% sequence identity with the CDRs of the exemplary antibodies 12D7, 12D7*, 31E4, 30D6, 15B12, 22A1, 1H12, 10E1 or 1D4.

In particular, these antibodies include the above-mentioned specific NY-ESO-1 antibodies which have been characterized *inter alia* by SEQ ID Nos. 1 to 86.

All of these specific individual NY-ESO-1 binding antibodies or fragments thereof binding NY-ESO-1 have in common that they have either been directly obtained from patients which have received a NY-ESO-1 vaccination and which have been classified as complete or at least partial responders or that they have been derived from antibodies of such patients. They thus are either monoclonal human patient-derived antibodies or monoclonal chimeric, humanized or human antibodies which preserve the essential properties of the monoclonal human patient-derived antibodies. It seems justified to assume that such antibodies will be particularly effective in the treatment of NY-ESO-1 expressing tumors or even other cancer types. The effectiveness of such antibodies may result from their capability to induce an immune response against the tumor by e.g. activating CD4⁺, CD8⁺ cytotoxic T cells.

The present disclosure further relates to nucleic acid molecules encoding for such antibodies, to nucleic acid molecules encoding for the variable light and/or heavy chains thereof and to nucleic acid molecules encoding for the CDR1, CDR2 and/or CDR3 of the variable light and/or heavy chains thereof.

The present disclosure further relates to vectors comprising such nucleic acid molecules and/or such vectors.

The present disclosure also relates to pharmaceutical compositions comprising such specific NY-ESO-1 binding antibodies or binding fragments thereof.

The present disclosure further relates to pharmaceutical compositions comprising such specific NY-ESO-1 binding antibodies or binding fragments thereof for use in treating hyper-proliferative disease, in particular tumors which express NY-ESO-1.

The present disclosure further relates to the use of such specific NY-ESO-1 binding antibodies or binding fragments thereof in the manufacture of a medicament for treating hyper-proliferative disease, in particular tumors which express NY-ESO-1.

The present disclosure further relates to methods of treating hyper-proliferative disease, in particular tumors which express NY-ESO-1 by administering to patients such specific NY-ESO-1 binding antibodies or binding fragments thereof.

The present disclosure further relates to a diagnostic composition comprising such specific NY-ESO-1 binding antibodies or binding fragments thereof for use in diagnosing hyper-proliferative diseases, in particular tumors which express NY-ESO-1.

The present disclosure further relates to the use of such specific NY-ESO-1 binding antibodies or binding fragments thereof in the manufacture of a diagnostic composition for diagnosing hyper-proliferative diseases, in particular tumors which express NY-ESO-1.

The present disclosure further relates to methods of diagnosing hyper-proliferative diseases, in particular tumors which express NY-ESO-1 by using such specific NY-ESO-1 binding antibodies or binding fragments thereof.

Antibodies or binding fragments thereof as far as they are generally referred to in the context of the present invention may also be part of larger immunoadhesion molecules, formed by covalent or non-covalent association of the antibody or antibody portion with e.g. one or more other proteins or peptides. Examples of such immunoadhesion molecules include use of the streptavidin core region to make a tetrameric scFv molecule (Kipriyanov, S. M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S. M., et al. (1994) Mol. Immunol. 31:1047-1058). Antibodies and fragments comprising immunoadhesion molecules can be obtained using standard recombinant DNA techniques, as described herein. Preferred antigen binding portions are complete domains or pairs of complete domains.

The binding antibodies and binding fragments of the present invention may also encompass domain antibody (dAb) fragments (Ward et al., Nature 341:544-546, 1989) which consist of a V_{H} domain. The antibodies and binding fragments of the present invention also encompass diabodies are bivalent antibodies in which V_{H} and V_{L} domains are expressed on a single polypeptide chain, but using a linker that is too short to allow for pairing between the two domains on the same chain, thereby forcing the domains to pair with complementary domains of another chain and creating two antigen binding sites (see e.g., EP 404,097; WO 93/11161; Holliger et al., Proc. Natl. Acad. Sci. USA 90:6444-6448, 1993, and Poljak et al., Structure 2:1121-1123, 1994). Diabodies can be bispecific or monospecific.

As mentioned the antibodies and binding fragments of the present invention also encompass single-chain antibody fragments (scFv). An scFv comprises an antibody heavy chain variable region (V_{H}) operably linked to an antibody light chain variable region (V_{L}) wherein the heavy chain variable region and the light chain variable region, together or individually, form a binding site. A scFv may comprise a V_{H} region at the amino-terminal end and a V_{L} region at the carboxy-terminal end. Alternatively, scFv may comprise a V_{L} region at the amino-terminal end and a V_{H} region at the carboxy-terminal end. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883).

A scFv may optionally further comprise a polypeptide linker between the heavy chain variable region and the light chain variable region. Such polypeptide linkers generally comprise between 1 and 50 amino acids, alternatively between 3 and 12 amino acids, alternatively 2 amino acids. An example of a linker peptide for linking heavy and light chains in a scFv comprises the 5 amino acid sequence Gly-Gly-Gly-Gly-Ser (SEQ ID NO:37). Other examples comprise one or more tandem repeats of this sequence (for example, a polypeptide comprising two to four repeats of Gly-Gly-Gly-Gly-Ser (SEQ ID NO:37)) to create linkers.

The antibodies and binding fragments of the present invention also encompass heavy chain antibodies (HCAb). Exceptions to the H₂L₂ structure of conventional antibodies occur in some isotypes of the immunoglobulins found in camelids (camels, dromedaries and llamas; Hamers-Casterman et al., 1993 Nature 363: 446; Nguyen et al., 1998 J. Mol. Biol. 275: 413), wobbegong sharks (Nuttall et al., Mol Immunol. 38:313-26, 2001), nurse sharks (Greenberg et al., Nature 374:168-73, 1995; Roux et al., 1998 Proc. Nat. Acad. Sci. USA 95: 11804), and in the spotted ratfish (Nguyen, et al., "Heavy-chain antibodies in Camelidae; a case of evolutionary innovation," 2002 Immunogenetics 54(1): 39-47). These antibodies can apparently form antigen-binding regions using only heavy chain variable region, in that these functional antibodies are dimers of heavy chains only (referred to as "heavy-chain antibodies" or "HCAbs"). Accordingly, some embodiments of the present antibodies and binding fragments may be heavy chain antibodies (HCAb) that specifically bind to the tumor associated antigen. For example, heavy chain antibodies that are a class of IgG and devoid of light chains are produced by animals of the genus *Camelidae* which includes camels, dromedaries and llamas (Hamers-Casterman et al., Nature 363:446-448 (1993)). HCAbs have a molecular weight of about 95 kDa instead of the about 160 kDa molecular weight of conventional IgG antibodies. Their binding domains consist only of the heavy-chain variable domains, often referred to as V_{HH} to distinguish them from conventional V_{H}. Muyldermans et al., J. Mol. Recognit. 12:131-140 (1999). The variable domain of the heavy-chain antibodies is sometimes referred to as a nanobody (Cortez-Retamozo et al., Cancer Research 64:2853-57, 2004). A nanobody library may be generated from an immunized dromedary as described in Conrath et al., (Antimicrob Agents Chemother 45: 2807-12, 2001) or using recombinant methods.

Since the first constant domain (C_{H1}) is absent (spliced out during mRNA processing due to loss of a splice consensus signal), the variable domain (V_{HH}) is immediately followed by the hinge region, the C_{H2} and the C_{H3} domains (Nguyen et al., Mol. Immunol. 36:515-524 (1999); Woolven et al., Immunogenetics 50:98-101 (1999)). Camelid V_{HH} reportedly recombines with IgG2 and IgG3 constant regions that contain hinge, CH2, and CH3 domains and lack a CH1 domain (Hamers-Casterman *et al., supra*). For example, llama IgG1 is a conventional (H₂L₂) antibody isotype in which V_{H} recombines with a constant region that contains hinge, CH1, CH2 and CH3 domains, whereas the llama IgG2 and IgG3 are heavy chain-only isotypes that lack CH1 domains and that contain no light chains.

Although the HCAbs are devoid of light chains, they have an antigen-binding repertoire. The genetic generation mechanism of HCAbs is reviewed in Nguyen et al. Adv. Immunol 79:261-296 (2001) and Nguyen et al., Immunogenetics 54:39-47 (2002). Sharks, including the nurse shark, display similar antigen receptor-containing single monomeric V-domains. Irving et al., J. Immunol. Methods 248:31-45 (2001); Roux et al., Proc. Natl. Acad. Sci. USA 95:11804 (1998).

V_{HH}s comprise small intact antigen-binding fragments (for example, fragments that are about 15 kDa, 118-136 residues). Camelid V_{HH} domains have been found to bind to antigen with high affinity (Desmyter et al., J. Biol. Chem. 276:26285-90, 2001), with V_{HH} affinities typically in the nanomolar range and comparable with those of Fab and scFv fragments. V_{HH}s are highly soluble and more stable than the corresponding derivatives of scFv and Fab fragments. V_{H} fragments have been relatively difficult to produce in soluble form, but improvements in solubility and specific binding can be obtained when framework residues are altered to be more V_{HH}-like. (See, for example, Reichman et al., J Immunol Methods 1999, 231:25-38.) V_{HH}s carry amino acid substitutions that make them more hydrophilic and prevent prolonged interaction with BiP (Immunoglobulin heavy-chain binding protein), which normally binds to the H-chain in the Endoplasmic Reticulum (ER) during folding and assembly, until it is displaced by the L-chain. Because of the V_{HH}s' increased hydrophilicity, secretion from the ER is improved.

Functional V_{HH}s may be obtained by proteolytic cleavage of HCAb of an immunized camelid, by direct cloning of V_{HH} genes from B-cells of an immunized camelid resulting in recombinant V_{HH}s, or from naive or synthetic libraries. V_{HH}s with desired antigen specificity may also be obtained through phage display methodology. Using V_{HH}s in phage display is much simpler and more efficient compared to Fabs or scFvs, since only one domain needs to be cloned and expressed to obtain a functional antigen-binding fragment. Muyldermans, Biotechnol. 74:277-302 (2001); Ghahroudi et al., FEBS Lett. 414:521-526 (1997); and van der Linden et al., J. Biotechnol. 80:261-270 (2000). Methods for generating antibodies having camelid heavy chains are also described in U.S. Patent Publication Nos. 20050136049 and 20050037421.

The binding antibodies and binding fragments thereof may also encompass any of the e.g. foregoing specifically mentioned amino acid sequences of the light or heavy chains with one or more conservative substitutions (e.g., 1, 2, 3, 4, 5, 6, 7, 8, 9, 10, 11, 12, 13, 14, or 15 conservative substitutions). One can determine the positions of an amino acid sequence that are candidates for conservative substitutions, and one can select synthetic and naturally-occurring amino acids that effect conservative substitutions for any particular amino acids. Consideration for selecting conservative substitutions include the context in which any particular amino acid substitution is made, the hydrophobicity or polarity of the side-chain, the general size of the side chain, and the pK value of side-chains with acidic or basic character under physiological conditions. For example, lysine, arginine, and histidine are often suitably substituted for each other. As is known in the art, this is because all three amino acids have basic side chains, whereas the pK value for the side-chains of lysine and arginine are much closer to each other (about 10 and 12) than to histidine (about 6). Similarly, glycine, alanine, valine, leucine, and isoleucine are often suitably substituted for each other, with the proviso that glycine is frequently not suitably substituted for the other members of the group. Other groups of amino acids frequently suitably substituted for each other include, but are not limited to, the group consisting of glutamic and aspartic acids; the group consisting of phenylalanine, tyrosine, and tryptophan; and the group consisting of serine, threonine, and, optionally, tyrosine.

By making conservative modifications to the amino acid sequence or corresponding modifications to the encoding nucleotides, one can produce antibodies or binding fragments thereof having functional and chemical characteristics similar to those of the exemplary antibodies and fragments disclosed herein.

The binding antibodies and binding fragments thereof as they are mentioned in the context of the present invention may encompass derivatives of the exemplary antibodies, fragments and sequences disclosed herein. Derivatives include polypeptides or peptides, or variants, fragments or derivatives thereof, which have been chemically modified. Examples include covalent attachment of one or more polymers, such as water soluble polymers, N-linked, or O-linked carbohydrates, sugars, phosphates, and/or other such molecules such as detectable labels such as fluorophores.

Labeling agents may be coupled either directly or indirectly to the antibodies or antigens of the invention. One example of indirect coupling is by use of a spacer moiety. Furthermore, the antibodies of the present invention can comprise a further domain, said domain being linked by covalent or noncovalent bonds. The linkage can be based on genetic fusion according to the methods known in the art and described above or can be performed by, e.g., chemical cross-linking as described in, e.g., international application WO 94/04686. The additional domain present in the fusion protein comprising the antibody of the invention may preferably be linked by a flexible linker, advantageously a polypeptide linker, wherein said polypeptide linker comprises plural, hydrophilic, peptide-bonded amino acids of a length sufficient to span the distance between the C-terminal end of said further domain and the N-terminal end of the antibody of the invention or vice versa. The therapeutically or diagnostically active agent can be coupled to the antibody of the invention or an antigen-binding fragment thereof by various means. This includes, for example, single-chain fusion proteins comprising the variable regions of the antibody of the invention coupled by covalent methods, such as peptide linkages, to the therapeutically or diagnostically active agent. Further examples include molecules which comprise at least an antigen-binding fragment coupled to additional molecules covalently or non-covalently include those in the following non-limiting illustrative list. Traunecker et al., Int. J. Cancer Surp. SuDP 7 (1992), 51-52, describe the bispecific reagent janusin in which the Fv region directed to CD3 is coupled to soluble CD4 or to other ligands such as OVCA and IL-7. Similarly an Fv region directed to NY-ESO-1 may be coupled to portions of e.g. an anti-CD40 agonistic antibody and/or portions of an anti-CTLA4 antagonistic antibody. Similarly, the variable regions of the antibody of the invention can be constructed into Fv molecules and coupled to alternative ligands such as those illustrated in the cited article. Higgins et al., J. Infect Disease 166 (1992), 198-202, described a hetero-conjugated antibody composed of OKT3 cross-linked to an antibody directed to a specific sequence in the V3 region of GP120. Such hetero-conjugate antibodies can also be constructed using at least the variable regions contained in the antibody of the invention methods. Additional examples of specific antibodies include those described by Fanger et al., Cancer Treat. Res. 68 (1993), 181-194 and by Fanger et al., Crit. Rev. Immunol. 12 (1992), 101-124. Conjugates that are immunotoxins including conventional antibodies have been widely described in the art. The toxins may be coupled to the antibodies by conventional coupling techniques or immunotoxins containing protein toxin portions can be produced as fusion proteins. The antibodies of the present invention can be used in a corresponding way to obtain such immunotoxins. Illustrative of such immunotoxins are those described by Byers et al., Seminars Cell. Biol. 2 (1991), 59-70 and by Fanger et al., Immunol. Today 12 (1991), 51-54.

The above described fusion proteins may further comprise a cleavable linker or cleavage site for proteases. These spacer moieties, in turn, can be either insoluble or soluble (Diener et al., Science 231 (1986), 148) and can be selected to enable drug release from the antigen at the target site.

Examples of therapeutic agents which can be coupled to the antibodies and antigens of the present invention for immunotherapy are drugs, radioisotopes, lectins, and toxins. The drugs with which can be conjugated to the antibodies and antigens of the present invention include compounds which are classically referred to as drugs such as mitomycin C, daunorubicin, and vinblastine. In using radioisotopically conjugated antibodies or antigens of the invention for, e.g., tumor immunotherapy, certain isotopes may be more preferable than others depending on such factors as leukocyte distribution as well as stability and emission.

Some emitters may be preferable to others. In general, alpha and beta particle emitting radioisotopes are preferred in immunotherapy. Preferred are short range, high energy a emitters such as ²¹²Bi. Examples of radioisotopes which can be bound to the antibodies or antigens of the invention for therapeutic purposes are ¹²⁵I, ¹³¹I, ⁹⁰Y, ⁶⁷Cu, ²¹²Bi, ²¹²At, ²¹¹Pb, ⁴⁷Sc, ¹⁰⁹Pd and ¹⁸⁸Re. Other therapeutic agents which can be coupled to the antibody or antigen of the invention, as well as ex vivo and in vivo therapeutic protocols, are known, or can be easily ascertained, by those of ordinary skill in the art.

As mentioned, the disclosure also relates in some embodiment to nucleic acid molecules encoding antibodies and binding fragments thereof, vectors comprising such nucleic acid molecules and host cells comprising such nucleic acid sequences and vectors.

The antibodies and binding fragments thereof may be encoded by a single nucleic acid (e.g., a single nucleic acid comprising nucleotide sequences that encode the light and heavy chain polypeptides of the antibody), or by two or more separate nucleic acids, each of which encode a different part of the antibody or antibody fragment. In this regard, the disclosure provides one or more nucleic acids that encode any of the forgoing antibodies, or binding fragments (e.g., any of the foregoing light or heavy chain variable regions of .SEQ ID NOs: 4, 14, 18, 30, 40, 50, 60, 70, 80 or SEQ ID Nos.: 3, 13, 187, 29, 39, 49, 59, 69, 79 or any of the CDRs of SEQ ID Nos.: 8, 22, 34, 44, 54, 64, 74, 84, 9, 23, 35, 45, 55, 65, 75, 85, 10, 24, 36, 46, 56, 66, 76, 86 or SEQ ID Nos.: 5, 19, 31, 41, 51, 61, 71, 81, 6, 20, 32, 42, 52, 62, 72, 82, 7, 21, 33, 43, 53, 63, 73, 83). The nucleic acid molecules may be DNA, cDNA, RNA and the like.

According to one aspect of the disclosure, the disclosure provides a nucleic acid that encodes a heavy chain variable region of an antibody or a portion thereof. Exemplary nucleic acid sequences are provided in SEQ ID Nos: 1, 11, 15, 25, 26, 37, 47, 57, 67, 77. The disclosure also provides a nucleic acid that encodes a light chain variable region of an antibody or a portion thereof. Exemplary nucleic acid sequences are provided in SEQ ID Nos.: 2, 12, 16, 27, 28, 38, 48, 58, 68, 78.

Also encompassed by the invention are nucleic acids encoding any of the foregoing amino acid sequences of the light or heavy chains that comprise one or more conservative substitutions (e.g., 1, 2, 3, 4, 5, 6, 7, 8, 9, 10, 11, 12, 13, 14, or 15 conservative substitutions), as discussed with respect to the antibody and antibody fragment of the invention, where the antibody or fragment comprising the substitution has the same or substantially the same affinity and specificity of epitope binding as one or more of the exemplary antibodies, fragments and sequences disclosed herein.

Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally associated or heterologous promoter regions.

The nucleic acids described herein can be inserted into vectors, e.g., nucleic acid expression vectors and/or targeting vectors. Such vectors can be used in various ways, e.g., for the expression of an antibody or a binding fragment in a cell or transgenic animal. Accordingly, the invention provides a vector comprising any one or more of the nucleic acids of the invention. A "vector" is any molecule or composition that has the ability to carry a nucleic acid sequence into a suitable host cell where synthesis of the encoded polypeptide can take place. Typically and preferably, a vector is a nucleic acid that has been engineered, using recombinant DNA techniques that are known in the art, to incorporate a desired nucleic acid sequence (e.g., a nucleic acid of the invention). Desirably, the vector is comprised of DNA. However, vectors that are not based on nucleic acids, such as liposomes, are also known in the art and can be used in connection with the invention. The inventive vector can be based on a single type of nucleic acid (e.g., a plasmid) or non-nucleic acid molecule (e.g., a lipid or a polymer). Alternatively, the vector can be a combination of a nucleic acid and a non-nucleic acid (i.e., a "chimeric" vector). For example, a plasmid harboring the nucleic acid can be formulated with a lipid or a polymer as a delivery vehicle. Such a vector is referred to herein as a "plasmid-lipid complex" and a "plasmid-polymer" complex, respectively. The inventive gene transfer vector can be integrated into the host cell genome or can be present in the host cell in the form of an episome.

Vectors are typically selected to be functional in the host cell in which the vector will be used (the vector is compatible with the host cell machinery such that amplification of the gene and/or expression of the gene can occur). A nucleic acid molecule encoding an antibody or binding fragment thereof may be amplified/expressed in prokaryotic, yeast, insect (baculovirus systems) and/or eukaryotic host cells. Selection of the host cell will depend in part on whether the antibody or fragment is to be post-transitionally modified (e.g., glycosylated and/or phosphorylated). If so, yeast, insect, or mammalian host cells are preferable.

Expression vectors typically contain one or more of the following components (if they are not already provided by the nucleic acid molecules): a promoter, one or more enhancer sequences, an origin of replication, a transcriptional termination sequence, a complete intron sequence containing a donor and acceptor splice site, a leader sequence for secretion, a ribosome binding site, a polyadenylation sequence, a polylinker region for inserting the nucleic acid encoding the polypeptide to be expressed, and a selectable marker element.

The invention in some aspects further provides a cell (e.g., an isolated or purified cell) comprising a nucleic acid or vector of the invention. The cell can be any type of cell capable of being transformed with the nucleic acid or vector of the invention so as to produce a polypeptide encoded thereby. The cell is preferably the cell of a mammal, such as a human, and is more preferably a hybridoma cell, an embryonic stem cell, or a fertilized egg. The embryonic stem cell or fertilized egg may not be a human embryonic stem cell or a human fertilized egg.

The host cells may be prokaryotic host cells (such as E. coli) or eukaryotic host cells (such as a yeast cell, an insect cell, or a vertebrate cell). The host cell, when cultured under appropriate conditions, expresses an antibody or binding fragment which can subsequently be collected from the culture medium (if the host cell secretes it into the medium) or directly from the host cell producing it (if it is not secreted). Selection of an appropriate host cell will depend upon various factors, such as desired expression levels, polypeptide modifications that are desirable or necessary for activity, such as glycosylation or phosphorylation, and ease of folding into a biologically active molecule. A number of suitable host cells are known in the art and many are available from the American Type Culture Collection (ATCC), Manassas, Va. Examples include mammalian cells, such as Chinese hamster ovary cells (CHO) (ATCC No. CCL61) CHO DHFR-cells (Urlaub et al. Proc. Natl. Acad. Sci. USA 97, 4216-4220 (1980)), human embryonic kidney (HEK) 293 or 293T cells (ATCC No. CRL1573), 3T3 cells (ATCC No. CCL92), or PER.C6 cells.

The cell comprising the nucleic acid or vector of the invention can be used to produce the antibody or binding fragment thereof, or a portion thereof (e.g., a heavy chain sequence, or a light chain sequence encoded by the nucleic acid or vector). After introducing the nucleic acid or vector of the invention into the cell, the cell is cultured under conditions suitable for expression of the encoded sequence. The antibody, antigen binding fragment, or portion of the antibody then can be isolated from the cell.

The TAA binding antibodies or binding fragments thereof as well as the compounds capable of activating the immune system can be formulated in compositions, especially pharmaceutical compositions. Such compositions comprise a therapeutically or prophylactically effective amount of an antibody or binding fragment thereof and/or of compounds capable of activating the immune system in admixture with a suitable carrier, e.g., a pharmaceutically acceptable agent.

Pharmaceutically acceptable agents for use in the present pharmaceutical compositions include carriers, excipients, diluents, antioxidants, preservatives, coloring, flavoring and diluting agents, emulsifying agents, suspending agents, solvents, fillers, bulking agents, buffers, delivery vehicles, tonicity agents, cosolvents, wetting agents, complexing agents, buffering agents, antimicrobials, and surfactants.

The composition can be in liquid form or in a lyophilized or freeze-dried form and may include one or more lyoprotectants, excipients, surfactants, high molecular weight structural additives and/or bulking agents (see for example US Patents 6,685,940, 6,566,329, and 6,372,716).

Compositions can be suitable for parenteral administration. Exemplary compositions are suitable for injection or infusion into an animal by any route available to the skilled worker, such as intraarticular, subcutaneous, intravenous, intramuscular, intraperitoneal, intracerebral (intraparenchymal), intracerebroventricular, intramuscular, intraocular, intraarterial, or intralesional routes. A parenteral formulation typically will be a sterile, pyrogen-free, isotonic aqueous solution, optionally containing pharmaceutically acceptable preservatives.

Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringers' dextrose, dextrose and sodium chlorine, lactated Ringer's, or fixed oils. Intravenous vehicles include fluid and nutrient replenishers, electrolyte replenishers, such as those based on Ringer's dextrose, and the like. Preservatives and other additives may also be present, such as, for example, anti-microbials, antioxidants, chelating agents, inert gases and the like. See generally, Remington's Pharmaceutical Science, 16th Ed., Mack Eds., 1980, which is incorporated herein by reference.

Pharmaceutical compositions described herein can be formulated for controlled or sustained delivery in a manner that provides local concentration of the product (e.g., bolus, depot effect) and/or increased stability or half-life in a particular local environment. The compositions can include the formulation of antibodies, binding fragments, nucleic acids, or vectors of the invention with particulate preparations of polymeric compounds such as polylactic acid, polyglycolic acid, etc., as well as agents such as a biodegradable matrix, injectable microspheres, microcapsular particles, microcapsules, bioerodible particles beads, liposomes, and implantable delivery devices that provide for the controlled or sustained release of the active agent which then can be delivered as a depot injection.

Both biodegradable and non-biodegradable polymeric matrices can be used to deliver compositions of the present invention, and such polymeric matrices may comprise natural or synthetic polymers. Biodegradable matrices are preferred. The period of time over which release occurs is based on selection of the polymer. Typically, release over a period ranging from between a few hours and three to twelve months is most desirable.

Alternatively or additionally, the compositions can be administered locally via implantation into the affected area of a membrane, sponge, or other appropriate material on to which an antibody, binding fragment, nucleic acid, or vector of the invention has been absorbed or encapsulated. Where an implantation device is used, the device can be implanted into any suitable tissue or organ, and delivery of an antibody, binding fragment, nucleic acid, or vector of the invention can be directly through the device via bolus, or via continuous administration, or via catheter using continuous infusion.

A pharmaceutical composition comprising a binding antibody or binding fragment thereof and/or compounds capable of activating the immune system can be formulated for inhalation, such as for example, as a dry powder. Inhalation solutions also can be formulated in a liquefied propellant for aerosol delivery. In yet another formulation, solutions may be nebulized.

Certain formulations containing antibodies or binding fragments thereof and/or compounds capable of activating the immune system can be administered orally. Formulations administered in this fashion can be formulated with or without those carriers customarily used in the compounding of solid dosage forms such as tablets and capsules. For example, a capsule can be designed to release the active portion of the formulation at the point in the gastrointestinal tract when bioavailability is maximized and pre-systemic degradation is minimized. Additional agents can be included to facilitate absorption of a selective binding agent. Diluents, flavorings, low melting point waxes, vegetable oils, lubricants, suspending agents, tablet disintegrating agents, and binders also can be employed.

The invention is now described with respect to some examples which are however not be construed as limiting.

### Example 1: Co-administration of CD40 agonistic antibody with human monoclonal antibody anti-NY-ESO-1 (12D7) and 5-FU

### Materials and Methods

### Syngeneic mousse tumour model CT26

1x10⁶ CT26/NY-ESO-1 colon carcinoma cells stably transfected with a human-NY-ESO-1 full length expression construct (cell line obtained from H. Nishikawa, Mie University, Mie, Japan) were inoculated s.c. into the flank of a hind limb of 8-10 week old Balb/C mice.

### Chemotherapy:

5-FU was administered i.p. at 75/mg/kg/injection.

### Antibodies:

Anti-human-NY-ESO-1 human monoclonal antibody 12D7 IgG1/kappa having a variable light chain of SEQ ID No.: 4 and a variable heavy chain of SEQ ID No.: 3 (see also WO 2008/110372 A1) was recombinantly expressed in 293HEK cells or CHO cells and purified using proteinA-sepharose. Dosing: 100ug/mouse/injection

Anti-murine CD40-agonistic rat IgG, 2a monoclonal antibody FGK45.5 was purified from hybridoma using proteinG-sepharose. The antibody was obtained from T. Rolink, Basel, Switzerland (see also Rolink et al., (1996) Immunity, 5(4), 319-330)

### Determination of tumor size:

In order to determine tumor size, the greatest longitudinal diameter (length) and the greatest transverse diameter (width) were determined using a caliper and the area was calculated.

### Experiment

Tumors were generated upon inoculation of CT26/NY-ESO-1 mouse colon carcinoma cells into Balb/C mice. Once the tumors reached a size of about 50 to 55 mm, a chemotherapy regimen using 5-FU was combined with the administration of anti-NY-ESO-1 12D7 antibody and CD40 agonistic antibody FGK45.5. The above mentioned dosages were applied. 5-FU was administered on day 14 and administration was repeated on day 21. Therapeutic antibodies were administered 2 days after chemotherapy to allow for access of antibody 12D7 to its intracellular target NY-ESO-1.

The combination of 5-FU plus 12D7 and CD40 agonistic antibody 45.5 resulted in a maximal reduction of tumor growth. The results are depicted in Fig. 1.

### Example 2: Administration of CTLA4 antagonistic antibody Ipilimumab in patient which has formed NY-ESO-1 binding antibodies after vaccination with NY-ESO-1.

A patient ZH311 was diagnosed in 2001 with metastatic melanoma. The tumor expressed NY-ESO-1 and was serum-positive for for NY-ESO-1..

In 2004 and 2005 this patient was vaccinated with NY-ESO-1 and showed a clinical response as demonstrated by regression of NY-Eso-1 positive liver metastases. Starting in 2007, the patient received treatment with Ipilimumab which lead to a stabilized course of disease The patient has experienced an overall survival time of 10 years which well exceeds the median survival time of 10 months within the peer group. Survival time after treatment with Ipilimumab was 3 years.

NY-ESO-1 binding antibody 12D7 was isolated from this patient as described in WO2008/110372A1.

The above observations together with the findings of example 1 suggest that a combination of an NY-ESO-1 binding antibody together with an anti-CTLA4 antagonistic antibody may have a positive clinical effect in therapy.

Some embodiments of the disclosure relate to:
1. Pharmaceutical composition comprising at least one tumor associated antigen (TAA) binding antibody or binding fragment thereof and at least one compound capable of activating the immune system.
2. Kit of pharmaceutical compositions comprising
   a) a first pharmaceutical composition comprising at least one tumor associated antigen (TAA) binding antibody or binding fragment thereof; and
   b) a second pharmaceutical composition comprising at least one compound capable of activating the immune system.
3. Pharmaceutical composition according to embodiment 1 or kit according to embodiment 2, wherein the at least one TAA binding antibody or binding fragment thereof binds to a CT antigen.
4. Pharmaceutical composition or kit according to any of embodiments 1 to 3, wherein the at least one TAA binding antibody or binding fragment thereof binds to a CT antigen selected from table 1 or 2.
5. Pharmaceutical composition or kit according to any of embodiments 1 to 4, wherein the at least one TAA binding antibody or binding fragment thereof is a monoclonal chimeric, humanized or human antibody or binding fragment thereof.
6. Pharmaceutical composition or kit according to any of embodiments 1 to 5, wherein the at least one TAA binding antibody or binding fragment thereof is a monoclonal human patient-derived antibody or binding fragment thereof.
7. Pharmaceutical composition or kit according to any of embodiments 1 to 6, wherein the at least one TAA binding antibody or binding fragment thereof comprises a constant region selected from the IgG class.
8. Pharmaceutical composition or kit according to any of embodiments 1 to 7, wherein the at least one TAA binding antibody or binding fragment thereof binds to the TAA with a Kd of about 0.1*10⁻¹² to about 1*10⁻⁶
9. Pharmaceutical composition or kit according to any of embodiments 1 to 8 wherein the TAA-antibody or binding fragment thereof and/or any other antibody or binding fragment thereof which is part of the pharmaceutical compositions or kits in accordance with any of embodiments 1 to 8 is coupled to a drug, a radioisotope, lectins, and/or a toxin.
10. Pharmaceutical composition or kit according to any of embodiments 1 to 9, wherein the at least one TAA binding antibody or binding fragment thereof binds to NY-ESO-1.
11. Pharmaceutical composition or kit according to any of embodiments 1 to 10, wherein the at least one TAA binding antibody or binding fragments thereof binds to NY-ESO-1 and is a patient-derived monoclonal human antibody or binding fragment thereof.
12. Pharmaceutical composition or kit according to any of embodiments 1 to 11, wherein the at least one TAA binding antibody or binding fragments thereof binds to NY-ESO-1 and comprises a light chain variable region and/or a heavy chain variable region, wherein
   a. the light chain variable region comprises at least a CDR1 selected from SEQ ID Nos.: 8, 22, 34, 44, 54, 64, 74, 84 or sequences at least 80% identical thereto, a CDR2 selected from SEQ ID Nos.: 9, 23, 35, 45, 55, 65, 75, 85 or sequences at least 80% identical thereto, and/or a CDR3 selected from SEQ ID Nos.: 10, 24, 36, 46, 56, 66, 76, 86 or sequences at least 80% identical thereto; and/or wherein
   b. the heavy chain variable region comprises at least a CDR1 selected from SEQ ID Nos.: 5, 19, 31, 41, 51, 61, 71, 81 or sequences at least 80% identical thereto, a CDR2 selected from SEQ ID Nos.: 6, 20, 32, 42, 52, 62, 72, 82 or sequences at least 80% identical thereto, and/or a CDR3 selected from SEQ ID Nos.: 7, 21, 33, 43, 53, 63, 73, 83 or sequences at least 80% identical thereto.
13. Pharmaceutical composition or kit according to embodiment 12, wherein the at least one TAA binding antibody or binding fragments thereof binds to NY-ESO-1 and comprises a light chain variable region and/or a heavy chain variable region, wherein
   a. the light chain variable region comprises at least a CDR1 selected from SEQ ID Nos.: 8, 22, 34, 44, 54, 64, 74, 84 or sequences at least 80% identical thereto, a CDR2 selected from SEQ ID Nos.: 9, 23, 35, 45, 55, 65, 75, 85 or sequences at least 80% identical thereto, and a CDR3 selected from SEQ ID Nos.: 10, 24, 36, 46, 56, 66, 76, 86 or sequences at least 80% identical thereto; and/or wherein
   b. the heavy chain variable region comprises at least a CDR1 selected from SEQ ID Nos.: 5, 19, 31, 41, 51, 61, 71, 81 or sequences at least 80% identical thereto, a CDR2 selected from SEQ ID Nos.: 6, 20, 32, 42, 52, 62, 72, 82 or sequences at least 80% identical thereto, and a CDR3 selected from SEQ ID Nos.: 7, 21, 33, 43, 53, 63, 73, 83 or sequences at least 80% identical thereto.
14. Pharmaceutical composition or kit according to any of embodiments 1 to 13, wherein the at least one TAA binding antibody or binding fragment thereof binds to NY-ESO-1 and wherein the antibody or binding fragment comprises a light chain variable region comprising SEQ ID Nos.: 4, 14, 18, 30, 40, 50, 60, 70, 80 or sequences at least 80% identical thereto and/or a heavy chain variable region comprising SEQ ID Nos.: 3, 13, 17, 29, 39, 49, 59, 69, 79 or sequences at least 80% identical thereto.
15. Pharmaceutical composition or kit according to embodiments 14, wherein the at least one TAA binding antibody or binding fragment thereof binds to NY-ESO-1 and wherein the antibody or binding fragment comprises a light chain variable region comprising SEQ ID Nos.: 4, 14, 18, 30, 40, 50, 60, 70, 80 or sequences at least 80% identical thereto and a heavy chain variable region comprising SEQ ID Nos.: 3, 13, 17, 29, 39, 49, 59, 69, 79 or sequences at least 80% identical thereto.
16. Pharmaceutical composition or kit according to any of embodiments 1 to 15, wherein the at least one compound capable of activating the immune system is selected from natural stimulants or at least co-stimulants of the immune system, agonistic activators of natural stimulants or at least co-stimulants of the immune system, or antagonistic effectors of natural inhibitors or at least co-inhibitors of the immune system.
17. Pharmaceutical composition or kit according to any of embodiments 1 to 16, wherein the at least one compound capable of activating the immune system is selected from CD40L, anti-CD40 agonistic antibodies, anti-OX40 agonistic antibodies, anti-CD 137 agonistic antibodies, anti-CTLA4 antagonistic antibodies, and anti-CD25 antagonistic antibodies.
18. Pharmaceutical composition or kit according to any of embodiments 1 to 17, wherein the at least one compound capable of activating the immune system is selected from CD40L, CP-870,893, SGN-40, Tremelimumab and Ipilimumab.
19. Pharmaceutical composition or kit according to any of embodiments 1 to 18, wherein the composition or the kit comprises at least two compounds capable of activating the immune system, of which the first compound is selected from natural stimulants or at least co-stimulants of the immune system or agonistic activators of natural stimulants or at least co-stimulants of the immune system, and of which the second compound is selected from antagonistic effectors of natural inhibitors or at least co-inhibitors of the immune system.
20. Pharmaceutical composition or kit according to embodiment 19, wherein the first compound capable of activating the immune system is selected from CD40L, anti-CD40 agonistic antibodies, anti-OX40 agonistic antibodies and anti-CD 137 agonistic antibodies and wherein the second compound capable of activating the immune system is selected from anti-CTLA4 antagonistic antibodies, and anti-CD25 antagonistic antibodies.
21. Pharmaceutical composition or kit according to embodiments 20, wherein the first compound capable of activating the immune system is selected from CD40L, CP-870,893 and SGN-40, and wherein the second compound capable of activating the immune system is selected from Tremelimumab and Ipilimumab.
22. Pharmaceutical composition according embodiment 1 or any of embodiments 3 to 21, wherein the at least one TAA binding antibody or binding fragment thereof and the at least one compound capable of activating the immune system take the form of a bi-specific antibody or binding fragment thereof.
23. Pharmaceutical composition according to embodiment 22, wherein the bi-specific antibody comprises (i) a TAA binding portion and (ii) a portion acting as agonistic activator of natural stimulants or at least co-stimulants of the immune system, or antagonistic effector of natural inhibitors or at least co-inhibitors of the immune system.
24. Pharmaceutical composition according to embodiment 23, wherein the bi-specific antibody comprises (i) a CT-antigen binding portion and (ii) a portion acting as anti-CD40 agonistic antibody, anti-OX40 agonistic antibody, anti-CD 137 agonistic antibody, anti-CTLA4 antagonistic antibody, or anti-CD25 antagonistic antibody.
25. Pharmaceutical composition according to embodiment 23, wherein the bi-specific antibody comprises (i) a NY-ESO-1 binding portion and (ii) a portion acting as anti-CD40 agonistic antibody or anti-CTLA4 antagonistic antibody.
26. Pharmaceutical composition according to any of embodiments 1 to 25, wherein the composition comprises additionally a cytotoxic agent.
27. Pharmaceutical composition according to embodiment 1 or any of embodiments 3 to 26, wherein the wherein the cytotoxic agent is selected from 5-fluoro-uracil, taxanes, anthracyclines, cisplatin, carboplatin, gemcitabine, capecitabin, navelbine or zoledronate.
28. Kit according to any of embodiments 2 to 21, wherein the kit comprises a third pharmaceutical composition comprising a cytotoxic agent.
29. Kit according to embodiment 28, wherein the cytotoxic agent is selected from 5-fluoro-uracil, taxanes, anthracyclines, cisplatin, carboplatin, gemcitabine, capecitabin, navelbine or zoledronate.
30. Pharmaceutical composition or kit according to any of embodiments 1 to 29 comprising a cytotoxic agent, a CT-antigen binding antibody or binding fragment thereof, and at least one compound selected from (i) natural stimulants or at least co-stimulants of the immune system, (ii) agonistic activators of natural stimulants or at least co-stimulants of the immune system and/or (iii) antagonistic effectors of natural inhibitors or at least co-inhibitors of the immune system.
31. Pharmaceutical composition or kit according to any of embodiments 1 to 30 comprising a cytotoxic agent, a CT-antigen binding antibody or binding fragment thereof, and at least one compound selected from agonistic activators of natural stimulants or at least co-stimulants of the immune system, or from antagonistic effectors of natural inhibitors or at least co-inhibitors of the immune system.
32. Pharmaceutical composition or kit according to any of embodiments 1 to 31 comprising a cytotoxic agent, a CT-antigen binding antibody or binding fragment thereof, at least one compound selected from agonistic activators of natural stimulants or at least co-stimulants of the immune system, and at least one compound selected from antagonistic effectors of natural inhibitors or at least co-inhibitors of the immune system.
33. Pharmaceutical composition or kit according to any of embodiments 29 to 32, wherein the CT-antigen binding antibody or binding fragments thereof recognizes NY-ESO-1, wherein the at least one compound selected from agonistic activators of natural stimulants or at least co-stimulants of the immune system is an anti-CD40 agonistic antibody and wherein the at least one compound selected from antagonistic effectors of natural inhibitors or at least co-inhibitors of the immune system is a anti-CTLA4 antagonistic antibody.
34. Combination of at least one tumor associated antigen (TAA) binding antibody or binding fragment thereof and at least one compound capable of activating the immune system for use in treating a patient wherein a TAA binding antibody or binding fragment thereof and at least one compound capable of activating the immune system is administered to the patient.
35. Combination for use as in embodiment 34, wherein a TAA binding antibody or binding fragment thereof and at least one compound capable of activating the immune system as mentioned in any of embodiments 3 to 25 is administered to the patient.
36. Combination for use as in embodiment 35, wherein the patient is subjected to cytotoxic treatment prior to, simultaneous with or subsequent to administration of said combination.
37. Combination for use as in embodiment 36, wherein the cytotoxic treatment includes chemotherapy, radiation therapy, surgery and/or hyperthermia.
38. Combination for use as in embodiment 37, wherein chemotherapy includes administration of agents selected from 5-fluoro-uracil, taxanes, anthracyclines, cisplatin, carboplatin, gemcitabine, capecitabin, navelbine or zoledronate.
39. Combination for use as in embodiments 34 to 38 for treating a hyper-proliferative disease.
40. Combination for use as in embodiment 39 for treating a hyper-proliferative disease which is characterized by expression of a TAA.
41. Combination for use as in embodiment 40, wherein said TAA is a CT antigen.
42. Combination for use as in embodiment 41, wherein said CT antigen is NY-ESO-1.
43. Combination for use as in any of embodiments 39 to 42, wherein said hyper-proliferative disease is selected from basal cell carcinoma; bladder cancer; bone cancer; central nervous system tumors; Burkitt's lymphoma; breast cancer; cervical cancer; chronic myelogenous leukemia; colon cancer; rectal cancer; colorectal cancer, esophageal cancer; Ewing family of tumors; extrahepatic bile duct cancer; gallbladder cancer; gastrointestinal stromal tumor (GIST); glioma; head and neck cancer; islet cell tumors; kaposi sarcoma; leukemia; liver cancer; lymphoma; Hodgkin's lymphoma; non-Hodgkin's lymphoma; mesothelioma; multiple myeloma/plasma cell neoplasm; myeloid leukemia; nasopharyngeal cancer; neuroblastoma; small cell lung cancer; non-small cell lung cancer; oropharyngeal cancer;; ovarian cancer; pancreatic cancer; parathyroid cancer; penile cancer; pharyngeal cancer; phaeochromocytoma; pituitary tumor; prostate cancer; renal cell (kidney) cancer; respiratory tract carcinoma; retinoblastoma; skin cancer (melanoma); small intestine cancer; soft tissue sarcoma; squamous cell carcinoma; squamous neck cancer; stomach (gastric) cancer; T-cell lymphoma; testicular cancer; throat cancer; thyroid cancer; transitional cell cancer of the renal pelvis and ureter; urethral cancer; uterine cancer; vaginal cancer; vulvar cancer and Wilms tumor.
44. Medicament for use in treating a patient wherein a pharmaceutical composition or a kit in accordance with any of embodiments 1 to 25 or a combination of at least one tumor associated antigen (TAA) binding antibody or binding fragment thereof and at least one compound capable of activating the immune system is administered to the patient.
45. Medicament for use as in embodiment 44, wherein the patient is subjected to cytotoxic treatment prior to, simultaneous with or subsequent to administration of a pharmaceutical composition or a kit in accordance with any of embodiments 1 to 25 or of a combination of at least one tumor associated antigen (TAA) binding antibody or binding fragment thereof and at least one compound capable of activating the immune system.
46. Medicament for use as in embodiment 46, wherein the cytotoxic treatment includes chemotherapy, radiation therapy, surgery and/or hyperthermia.
47. Medicament for use as in embodiment 47, wherein chemotherapy includes administration of agents selected from 5-fluoro-uracil, taxanes, anthracyclines, cisplatin, carboplatin, gemcitabine, capecitabin, navelbine or zoledronate.
48. Medicament for use as in embodiments 44 to 47 for treating a hyper-proliferative disease.
49. Medicament for use as in embodiment 48 for treating a hyper-proliferative disease which is characterized by expression of a TAA.
50. Medicament for use as in embodiment 49, wherein said TAA is a CT antigen.
51. Medicament for use as in embodiment 50, wherein said CT antigen is NY-ESO-1.
52. Medicament for use as in any of embodiments 48 to 51, wherein said hyper-proliferative disease is selected from basal cell carcinoma; bladder cancer; bone cancer; central nervous system tumors; Burkitt's lymphoma; breast cancer; cervical cancer; chronic myelogenous leukemia; colon cancer; rectal cancer; colorectal cancer, esophageal cancer; Ewing family of tumors; extrahepatic bile duct cancer; gallbladder cancer; gastrointestinal stromal tumor (GIST); glioma; head and neck cancer; islet cell tumors; kaposi sarcoma; leukemia; liver cancer; lymphoma; Hodgkin's lymphoma; non-Hodgkin's lymphoma; mesothelioma; multiple myeloma/plasma cell neoplasm; myeloid leukemia; nasopharyngeal cancer; neuroblastoma; small cell lung cancer; non-small cell lung cancer; oropharyngeal cancer;; ovarian cancer; pancreatic cancer; parathyroid cancer; penile cancer; pharyngeal cancer; phaeochromocytoma; pituitary tumor; prostate cancer; renal cell (kidney) cancer; respiratory tract carcinoma; retinoblastoma; skin cancer (melanoma); small intestine cancer; soft tissue sarcoma; squamous cell carcinoma; squamous neck cancer; stomach (gastric) cancer; T-cell lymphoma; testicular cancer; throat cancer; thyroid cancer; transitional cell cancer of the renal pelvis and ureter; urethral cancer; uterine cancer; vaginal cancer; vulvar cancer and Wilms tumor.
53. Medicament for use as in any of embodiments 44 to 52, wherein the at least one tumor associated antigen (TAA) binding antibody or binding fragment thereof and at least one compound capable of activating the immune system are as mentioned in any of embodiments 3 to 25.
54. Use of a pharmaceutical composition or a kit in accordance with any of embodiments 1 to 25 or a combination of at least one tumor associated antigen (TAA) binding antibody or binding fragment thereof and at least one compound capable of activating the immune system in the manufacture of a medicament for treating a patient.
55. Use as in embodiment 54, wherein the patient is subjected to cytotoxic treatment prior to, simultaneous with or subsequent to the administration of a pharmaceutical composition or a kit in accordance with any of embodiments 1 to 25 or of a combination of at least one tumor associated antigen (TAA) binding antibody or binding fragment thereof and at least one compound capable of activating the immune system.
56. Use as in embodiment 55, wherein the cytotoxic treatment includes chemotherapy, radiation therapy, surgery and/or hyperthermia.
57. Use as in embodiment 56, wherein chemotherapy includes administration of agents selected from 5-fluoro-uracil, taxanes, anthracyclines, cisplatin, carboplatin, gemcitabine, capecitabin, navelbine or zoledronate.
58. Use as in embodiments 54 to 47 for treating a hyper-proliferative disease.
59. Use as in embodiment 58 for treating a hyper-proliferative disease which is characterized by expression of a TAA.
60. Use as in embodiment 59, wherein said TAA is a CT antigen.
61. Use as in embodiment 60, wherein said CT antigen is NY-Eso-1.
62. Use as in any of embodiments 58 to 61, wherein said hyper-proliferative disease is selected from basal cell carcinoma; bladder cancer; bone cancer; central nervous system tumors; Burkitt's lymphoma; breast cancer; cervical cancer; chronic myelogenous leukemia; colon cancer; rectal cancer; colorectal cancer, esophageal cancer; Ewing family of tumors; extrahepatic bile duct cancer; gallbladder cancer; gastrointestinal stromal tumor (GIST); glioma; head and neck cancer; islet cell tumors; kaposi sarcoma; leukemia; liver cancer; lymphoma; Hodgkin's lymphoma; non-Hodgkin's lymphoma; mesothelioma; multiple myeloma/plasma cell neoplasm; myeloid leukemia; nasopharyngeal cancer; neuroblastoma; small cell lung cancer; non-small cell lung cancer; oropharyngeal cancer;; ovarian cancer; pancreatic cancer; parathyroid cancer; penile cancer; pharyngeal cancer; phaeochromocytoma; pituitary tumor; prostate cancer; renal cell (kidney) cancer; respiratory tract carcinoma; retinoblastoma; skin cancer (melanoma); small intestine cancer; soft tissue sarcoma; squamous cell carcinoma; squamous neck cancer; stomach (gastric) cancer; T-cell lymphoma; testicular cancer; throat cancer; thyroid cancer; transitional cell cancer of the renal pelvis and ureter; urethral cancer; uterine cancer; vaginal cancer; vulvar cancer and Wilms tumor.
63. Use as in any of embodiments 44 to 62, wherein the at least one tumor associated antigen (TAA) binding antibody or binding fragment thereof and at least one compound capable of activating the immune system are as mentioned in any of embodiments 3 to 25.
64. Method of treating a patient by administering a pharmaceutical composition or a kit in accordance with any of embodiments 1 to 25or a combination of at least one tumor associated antigen (TAA) binding antibody or binding fragment thereof and at least one compound capable of activating the immune system is administered to the patient.
65. Method as in embodiment 64, wherein the patient is subjected to cytotoxic treatment prior to, simultaneous with or subsequent to the administration of a pharmaceutical composition or a kit in accordance with any of embodiments 1 to 25 or of a combination of at least one tumor associated antigen (TAA) binding antibody or binding fragment thereof and at least one compound capable of activating the immune system.
66. Method as in embodiment 65, wherein the cytotoxic treatment includes chemotherapy, radiation therapy, surgery and/or hyperthermia.
67. Method as in embodiment 66, wherein chemotherapy includes administration of agents selected from 5-fluoro-uracil, taxanes, anthracyclines, cisplatin, carboplatin, gemcitabine, capecitabin, navelbine or zoledronate.
68. Method as in embodiments 64 to 67 for treating a hyper-proliferative disease.
69. Method as in embodiment 68 for treating a hyper-proliferative disease which is characterized by expression of a TAA.
70. Method as in embodiment 69, wherein said TAA is a CT antigen.
71. Method as in embodiment 70, wherein said CT antigen is NY-ESO-1.
72. Method as in any of embodiments 68 to 71, wherein said hyper-proliferative disease is selected from basal cell carcinoma; bladder cancer; bone cancer; central nervous system tumors; Burkitt's lymphoma; breast cancer; cervical cancer; chronic myelogenous leukemia; colon cancer; rectal cancer; colorectal cancer, esophageal cancer; Ewing family of tumors; extrahepatic bile duct cancer; gallbladder cancer; gastrointestinal stromal tumor (GIST); glioma; head and neck cancer; islet cell tumors; kaposi sarcoma; leukemia; liver cancer; lymphoma; Hodgkin's lymphoma; non-Hodgkin's lymphoma; mesothelioma; multiple myeloma/plasma cell neoplasm; myeloid leukemia; nasopharyngeal cancer; neuroblastoma; small cell lung cancer; non-small cell lung cancer; oropharyngeal cancer;; ovarian cancer; pancreatic cancer; parathyroid cancer; penile cancer; pharyngeal cancer; phaeochromocytoma; pituitary tumor; prostate cancer; renal cell (kidney) cancer; respiratory tract carcinoma; retinoblastoma; skin cancer (melanoma); small intestine cancer; soft tissue sarcoma; squamous cell carcinoma; squamous neck cancer; stomach (gastric) cancer; T-cell lymphoma; testicular cancer; throat cancer; thyroid cancer; transitional cell cancer of the renal pelvis and ureter; urethral cancer; uterine cancer; vaginal cancer; vulvar cancer and Wilms tumor.
73. Method as in any of embodiments 63 to 72, wherein the the at least one tumor associated antigen (TAA) binding antibody or binding fragment thereof and at least one compound capable of activating the immune system are as mentioned in any of embodiments 3 to 25.
74. Isolated monoclonal antibody or binding fragment thereof which binds to NY-ESO-1 and comprises a light chain variable region and/or a heavy chain variable region, wherein
   a. the light chain variable region comprises at least a CDR1 selected from SEQ ID Nos.: 8, 22, 34, 44, 54, 64, 74, 84 or sequences at least 80% identical thereto, a CDR2 selected from SEQ ID Nos.: 9, 23, 35, 45, 55, 65, 75, 85 or sequences at least 80% identical thereto, and/or a CDR3 selected from SEQ ID Nos.: 10, 24, 36, 46, 56, 66, 76, 86 or sequences at least 80% identical thereto; and/or wherein
   b. the heavy chain variable region comprises at least a CDR1 selected from SEQ ID Nos.: 5, 19, 31, 41, 51, 61, 71, 81 or sequences at least 80% identical thereto, a CDR2 selected from SEQ ID Nos.: 6, 20, 32, 42, 52, 62, 72, 82 or sequences at least 80% identical thereto, and/or a CDR3 selected from SEQ ID Nos.: 7, 21, 33, 43, 53, 63, 73, 83 or sequences at least 80% identical thereto.
75. Isolated monoclonal antibody or binding fragment thereof according to embodiment 74, which comprises a light chain variable region and/or a heavy chain variable region, wherein
   a. the light chain variable region comprises at least a CDR1 selected from SEQ ID Nos.: 8, 22, 34, 44, 54, 64, 74, 84 or sequences at least 80% identical thereto, a CDR2 selected from SEQ ID Nos.: 9, 23, 35, 45, 55, 65, 75, 85 or sequences at least 80% identical thereto, and a CDR3 selected from SEQ ID Nos.: 10, 24, 36, 46, 56, 66, 76, 86 or sequences at least 80% identical thereto; and/or wherein
   b. the heavy chain variable region comprises at least a CDR1 selected from SEQ ID Nos.: 5, 19, 31, 41, 51, 61, 71, 81 or sequences at least 80% identical thereto, a CDR2 selected from SEQ ID Nos.: 6, 20, 32, 42, 52, 62, 72, 82 or sequences at least 80% identical thereto, and a CDR3 selected from SEQ ID Nos.: 7, 21, 33, 43, 53, 63, 73, 83 or sequences at least 80% identical thereto.
76. Isolated monoclonal antibody or binding fragment thereof which binds to NY-ESO-1 and comprises a light chain variable region comprising SEQ ID Nos.: 4, 14, 18, 30, 40, 50, 60, 70, 80 or sequences at least 80% identical thereto and/or a heavy chain variable region comprising SEQ ID Nos.: 3, 13, 17, 29, 39, 49, 59, 69, 79 or sequences at least 80% identical thereto.
77. Isolated monoclonal antibody or binding fragment thereof according to embodiment 76, which comprises a light chain variable region comprising SEQ ID Nos.: 4, 14, 18, 30, 40, 50, 60, 70, 80 or sequences at least 80% identical thereto and a heavy chain variable region comprising SEQ ID Nos.: 3, 13, 17, 29, 39, 49, 59, 69, 79 or sequences at least 80% identical thereto.
78. Pharmaceutical composition comprising an antibody or binding fragment thereof of in accordance with any of embodiments 74 to 77.
79. Pharmaceutical composition comprising an antibody or binding fragment thereof of in accordance with any of embodiments 74 to 77 for use in the treatment of a hyper-proliferative disease.
80. Use of antibody or binding fragment thereof of in accordance with any of embodiments 74 to 77 in the manufacture of a medicament for treating a hyper-proliferative disease.
81. Method of treating a hyper-proliferative disease by administering to a patient in need thereof an antibody or binding fragment thereof of in accordance with any of embodiments 61 to 64 or a pharmaceutical composition in accordance with embodiment 64.
82. Pharmaceutical composition, use or method of any of embodiments 79 to 81, wherein the hyper-proliferative disease is characterized by expression of a TAA.
83. Pharmaceutical composition, use or method of embodiment 82, wherein said TAA is a CT antigen.
84. Pharmaceutical composition, use or method of embodiment 70, wherein said CT antigen is NY-ESO-1.
85. Pharmaceutical composition, use or method of any of embodiments 79 to 84, wherein said hyper-proliferative disease is selected from basal cell carcinoma; bladder cancer; bone cancer; central nervous system tumors; Burkitt's lymphoma; breast cancer; cervical cancer; chronic myelogenous leukemia; colon cancer; rectal cancer; colorectal cancer, esophageal cancer; Ewing family of tumors; extrahepatic bile duct cancer; gallbladder cancer; gastrointestinal stromal tumor (GIST); glioma; head and neck cancer; islet cell tumors; kaposi sarcoma; leukemia; liver cancer; lymphoma; Hodgkin's lymphoma; non-Hodgkin's lymphoma; mesothelioma; multiple myeloma/plasma cell neoplasm; myeloid leukemia; nasopharyngeal cancer; neuroblastoma; small cell lung cancer; non-small cell lung cancer; oropharyngeal cancer;; ovarian cancer; pancreatic cancer; parathyroid cancer; penile cancer; pharyngeal cancer; phaeochromocytoma; pituitary tumor; prostate cancer; renal cell (kidney) cancer; respiratory tract carcinoma; retinoblastoma; skin cancer (melanoma); small intestine cancer; soft tissue sarcoma; squamous cell carcinoma; squamous neck cancer; stomach (gastric) cancer; T-cell lymphoma; testicular cancer; throat cancer; thyroid cancer; transitional cell cancer of the renal pelvis and ureter; urethral cancer; uterine cancer; vaginal cancer; vulvar cancer and Wilms tumor.

## Claims

1. Pharmaceutical composition comprising at least one tumor associated antigen (TAA) binding antibody or binding fragment thereof and at least one anti-CD40 agonistic antibody or agonistic binding fragment thereof, wherein said at least one TAA binding antibody or binding fragment thereof binds to NY-ESO-1 and wherein said at least one TAA binding antibody or binding fragment thereof comprises a variable light chain region comprising a CDR1 of SEQ ID No.:8, a CDR2 of SEQ ID No.:9 and a CDR3 of SEQ ID No.: 10 and a variable heavy chain region comprising a CDR1 of SEQ ID No.:5, a CDR2 of SEQ ID No.:6 and a CDR3 of SEQ ID No.:7.

2. Kit of pharmaceutical compositions comprising
a) a first pharmaceutical composition comprising at least one tumor associated antigen (TAA) binding antibody or binding fragment thereof; and
b) a second pharmaceutical composition comprising at least one anti-CD40 agonistic antibody or agonistic binding fragment thereof,
wherein said at least one TAA binding antibody or binding fragment thereof binds to NY-ESO-1 and wherein said at least one TAA binding antibody or binding fragment
thereof comprises a variable light chain region comprising a CDR1 of SEQ ID No.:8, a CDR2 of SEQ ID No.:9 and a CDR3 of SEQ ID No.: 10 and a variable heavy chain region comprising a CDR1 of SEQ ID No.:5, a CDR2 of SEQ ID No.:6 and a CDR3 of SEQ ID No.:7.
DB: SPF

3. Pharmaceutical composition according to claim 1 or kit according to claim 2, wherein said at least one TAA binding antibody or binding fragment thereof binds to NY-ESO-1 and is a monoclonal chimeric, humanized or human antibody or binding fragment thereof.

4. Pharmaceutical composition or kit according to any of claims 1 to 3, wherein said at least one TAA binding antibody or binding fragment thereof binds to NY-ESO-1 and comprises a light chain variable region comprising SEQ ID No.: 4 and a heavy chain variable region comprising SEQ ID No.: 3.

5. Pharmaceutical composition according to any one of claims 1, 3 or 4, wherein said at least one TAA binding antibody or binding fragment thereof and said at least one anti-CD40 agonistic antibody or agonistic binding fragment thereof take the form of a bi-specific antibody.

6. Pharmaceutical composition according to any one of claims 1 or 3 to 5, wherein the composition comprises additionally a cytotoxic agent.

7. Kit according to any of claims 2 to 4, wherein the kit comprises a third pharmaceutical composition comprising a cytotoxic agent.

8. Medicament for use in treating a hyper-proliferative disease in a patient wherein a pharmaceutical composition in accordance with any of claims 1 or 3 to 6 is administered to the patient.

## Patentansprüche

1. Pharmazeutische Zusammensetzung umfassend mindestens einen ein tumor assoziiertes Antigen (TAA) bindenden Antikörper oder Bindungsfragment davon und mindestens einen anti-CD40 agonistischen Antikörper oder agonistisches Bindungsfragment davon, wobei dieser mindestens eine TAA bindende Antikörper oder Bindungsfragment davon an NY-ESO-1 bindet und wobei dieser mindestens eine TAA bindende Antikörper oder Bindungsfragment davon eine variable Region der leichten Kette umfassend eine CDR1 der SEQ ID No.:8, eine CDR2 der SEQ ID No.:9 und eine CDR3 der SEQ ID No.:10 und eine variable Region der schweren Kette umfassend eine CDR1 der SEQ ID No:5, eine CDR2 der SEQ ID No.:6 und eine CDR3 der SEQ ID No.:7, umfasst.

2. Kit pharmazeutischer Zusammensetzungen umfassend
a) eine erste pharmazeutische Zusammensetzung umfassend mindestens einen ein tumor assoziiertes Antigen (TAA) bindenden Antikörper oder Bindungsfragment davon; und
b) eine zweite pharmazeutische Zusammensetzung umfassend mindestens einen anti-CD40 agonistischen Antikörper oder agonistisches Bindungsfragment davon,
wobei dieser mindestens eine TAA bindende Antikörper oder Bindungsfragment davon an NY-ESO-1 bindet und wobei dieser mindestens eine TAA bindende Antikörper oder Bindungsfragment davon eine variable Region der leichten Kette umfassend eine CDR1 der SEQ ID No.:8, eine CDR2 der SEQ ID No.:9 und eine CDR3 der SEQ ID No.:10 und eine variable Region der schweren Kette umfassend eine CDR1 der SEQ ID No:5, eine CDR2 der SEQ ID No.:6 und eine CDR3 der SEQ ID No.:7, umfasst.

3. Pharmazeutische Zusammensetzung gemäß Anspruch 1 oder Kit gemäß Anspruch 2, wobei dieser mindestens eine TAA bindende Antikörper oder Bindungsfragment davon an NY-ESO-1 bindet und ein monoklonaler chimerischer, humanisierter oder humaner Antikörper oder Bindungsfragment davon ist.

4. Pharmazeutische Zusammensetzung oder Kit gemäß einem der Ansprüche 1 bis 3, wobei dieser mindestens eine TAA bindende Antikörper oder Bindungsfragment davon an NY-ESO-1 bindet und eine variable Region der leichten Kette umfassend SEQ ID No.:4 und eine variable Region der schweren Kette umfassend SEQ ID No.:3, umfasst.

5. Pharmazeutische Zusammensetzung gemäß einem der Ansprüche 1, 3 oder 4, wobei dieser mindestens eine TAA bindende Antikörper oder Bindungsfragment davon und dieser mindestens eine anti-CD40 agonistische Antikörper oder agonistisches Bindungsfragment davon die Form eines bispezifischen Antikörper annehmen.

6. Pharmazeutische Zusammensetzung gemäß einem der Ansprüche 1 oder 3 bis 5, wobei die Zusammensetzung zusätzlich einen zytotoxischen Wirkstoff umfasst.

7. Kit gemäß einem der Ansprüche 2 bis 4, wobei der Kit eine dritte pharmazeutische Zusammensetzung einen zytotoxischen Wirkstoff umfassend, umfasst.

8. Arzneimittel zur Verwendung bei der Behandlung einer hyper-proliferativen Krankheit eines Patienten, wobei dem Patienten eine pharmazeutische Zusammensetzung gemäß einem der Ansprüche 1 oder 3 bis 6 verabreicht wird.

## Revendications

1. Composition pharmaceutique comprenant au moins un anticorps ou un fragment de fixation de ce dernier se liant à un antigène associé à une tumeur (TAA) et au moins un anticorps agoniste anti CD 40 ou un fragment de fixation agoniste de celui-ci, où ledit au moins un anticorps de fixation sur un TAA ou un fragment de fixation de celui-ci se lie au NY-ESO-1 et où ledit au moins un anticorps de fixation à un TAA ou un fragment de fixation de celui-ci comprend un domaine variable de chaîne légère comprenant un CDR1 de SEQ ID No : 8, un CDR2 de SEQ ID No : 9 et un CDR3 de SEQ ID No : 10 et un domaine variable de chaîne lourde comprenant un CDR1 de SEQ TD No : 5, un CDR2 de SEQ ID No : 6 et un CDR3 de SEQ ID No : 7.

2. Kit de compositions pharmaceutiques comprenant :
a) une première composition pharmaceutique comprenant au moins un anticorps ou un fragment de fixation de celui-ci se liant à un antigène associé à une tumeur (TAA) ; et
b) une deuxième composition pharmaceutique comprenant au moins un anticorps agoniste anti CD 40 ou un fragment de fixation agoniste de celui-ci,
dans lequel ledit au moins un anticorps se liant à un TAA ou un fragment de fixation de celui-ci se lie au NY-ESO-1 et dans lequel ledit un anticorps de fixation à un TAA ou un fragment de fixation de celui-ci comprend un domaine variable de chaîne légère comprenant un CDR1 de SEQ ID No : 8, un CDR2 de SEQ ID No : 9 et un CDR3 de SEQ ID No : 10 et un domaine variable de chaîne lourde comprenant un CDR1 de SEQ TD No : 5, un CDR2 de SEQ ID No : 6 et un CDR3 de SEQ ID No : 7.

3. Composition pharmaceutique selon la revendication 1, ou kit selon la revendication 2, dans laquelle / lequel ledit au moins un anticorps se liant à un TAA ou un fragment de fixation de celui-ci se lie au NY-ESO-1 et est un anticorps monoclonal chimérique humanisé ou humain, ou un fragment de fixation de celui-ci.

4. Composition pharmaceutique ou kit selon l'une quelconque des revendications 1 à 3, dans laquelle / lequel au moins un anticorps se liant à un TAA ou un fragment de fixation de celui-ci se lie au NY-ESO-1 et comprend un domaine variable de chaîne légère comprenant la SEQ ID No : 4 et un domaine variable de chaîne lourde comprenant la SEQ ID No : 3.

5. Composition pharmaceutique selon l'une quelconque des revendications 1, 3 ou 4, dans laquelle ledit au moins un anticorps se liant à un TAA ou un fragment de fixation de celui-ci et ledit au moins un anticorps agoniste anti CD 40 ou un fragment de fixation agoniste de celui-ci prennent la forme d'un anticorps bispécifique.

6. Composition pharmaceutique selon l'une quelconque des revendications 1, ou 3 à 5, où la composition comprend à titre complémentaire un agent cytotoxique.

7. Kit selon l'une quelconque des revendications 2 à 4, où le kit comprend une troisième composition pharmaceutique comprenant un agent cytotoxique.

8. Médicament pour une utilisation dans le traitement d'une pathologie hyper proliférante chez un patient où une composition pharmaceutique en conformité avec l'une quelconque des revendications 1, ou 3 à 6, est administrée au patient.
